(19) Europäisches Patentamt
European Patent Office
Office européen des brevets

(11) EP 1 490 080 B1

(12) **EUROPEAN PATENT SPECIFICATION**

(45) Date of publication and mention of the grant of the patent:
**03.11.2010 Bulletin 2010/44**

(21) Application number: **03709488.5**

(22) Date of filing: **26.03.2003**

(51) Int Cl.:
***A61K 36/88*** *(2006.01)* ***A61P 9/10*** *(2006.01)*

(86) International application number:
**PCT/CA2003/000433**

(87) International publication number:
**WO 2003/080084 (02.10.2003 Gazette 2003/40)**

(54) **medical use of anthocyanins extracted from black rice**

medizinische Verwendung von Anthocyaninen aus Schwarzreis

Utilisation médicale d'anthocyanines du riz noir

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **26.03.2002 US 108305**

(43) Date of publication of application:
**29.12.2004 Bulletin 2004/53**

(73) Proprietor: **Forbes Medi-Tech Inc.**
**Vancouver, British Columbia V6C 2T8 (CA)**

(72) Inventors:
- **ZAWISTOWSKI, Jerzy**
**Port Moody, British Columbia V3H 4K6 (CA)**
- **HU, Chun**
**Vancouver, British Columbia V6T 1R9 (CA)**
- **KITTS, David, D.**
**Burnaby, British Columbia V5H 1P2 (CA)**

(74) Representative: **Jones, Helen M.M.**
**Gill Jennings & Every LLP**
**Broadgate House**
**7 Eldon Street**
**London EC2M 7LH (GB)**

(56) References cited:
- **SU NOH RYU ET AL: "High performance liquid chromatographic determination of anthocyanin pigments in some varieties of black rice." JOURNAL OF FOOD AND DRUG ANALYSIS 6 (4) 729-736 1998 CORRESPONDENCE (REPRINT) ADDRESS, CHI-TANG HO, DEP. OF FOOD SCI., COOK COLL., RUTGERS STATE UNIV., NEW BRUNSWICK, NJ 08901-8520, USA, XP001153058**
- **LING WEN HUA ET AL: "Supplementation of the black rice outer layer fraction to rabbits decreases atherosclerotic plaque formation and increases antioxidant status." THE JOURNAL OF NUTRITION. UNITED STATES JAN 2002, vol. 132, no. 1, January 2002 (2002-01), pages 20-26, XP002245979 ISSN: 0022-3166**
- **DATABASE FSTA [Online] INTERNATIONAL FOOD INFORMATION SERVICE (IFIS), FRANFURT/MAIN, DE; HEE JONG KOH ET AL: "Varietal variation of pigmentation and some nutritive characteristics of colored rices." Database accession no. 97-1-05-m0033 XP002245980 & KOREAN JOURNAL OF CROP SCIENCE 1996 COLL. OF AGRIC. & LIFE SCI., SEOUL NAT. UNIV., SUWON 441-744, KOREA, vol. 41, no. 5, pages 600-607,**
- **DATABASE BIOSIS [Online] BIOSCIENCES INFORMATION SERVICE, PHILADELPHIA, PA, US; 1995 YOON HYE-HYUN ET AL: "Identification of anthocyanins from Korean pigmented rice." Database accession no. PREV199698726462 XP002245981 & AGRICULTURAL CHEMISTRY AND BIOTECHNOLOGY, vol. 38, no. 6, 1995, pages 581-583, ISSN: 0368-2897**
- **ABDEL-AAL E S M ET AL: "A rapid method for quantifying total anthocyanins in blue aleurone and purple pericarp wheats." CEREAL CHEMISTRY 76 (3) 350-354 1999 CROP DEV. CENT., DEP. OF PLANT SCI., UNIV. OF SASKATCHEWAN, SASKATOON, SASK. S7N 5A8, CANADA. FAX 306/966-5015. E-MAIL ABDELAAL(A)SASK.USASK.CA, XP009013063**
- **SU NOH RYU ET AL: 'HPLC determiation of anthocyanin pigments in some varieties of black rice' J. OF FOOD AND DRUG ANALYSIS vol. 6, no. 4, 1998, pages 729 - 736, XP001153058**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **LING W.H. ET AL: 'Red and black rice decrease atherosclerotic plaque formation and increase antioxidant status in rabbits' J. NUTR. vol. 131, 2001, pages 1421 - 1426**

Remarks:

The file contains technical information submitted after the application was filed and not included in this specification

## Description

## BACKGROUND OF THE INVENTION

While recent advances in science and technology are helping to improve quality and add years to human life, the prevention of atherosclerosis, an underlying cause of cardiovascular disease ("CVD") has not been sufficiently addressed and it remains the leading cause of death in the United states, Europe and part of Asia (1). Atherosclerosis is a degenerative process resulting from an interplay of inherited (genetic) factors and environmental factors such as diet and lifestyle. Research to date suggest that cholesterol may play a role in atherosclerosis by forming atherosclerotic plaques in blood vessels, ultimately cutting off blood supply to the heart muscle or alternatively to the brain or limbs, depending on the location of the plaque in the arterial tree (2,3). Overviews have indicated that a 1% reduction in an individual's total serum cholesterol yields a 2% reduction in risk of a coronary artery event (4). Statistically, a 10% decrease in average serum cholesterol (e.g. from 6.0 mmol/L to 5.3 mmol/L) may result in the prevention of 100,000 deaths in the United States annually (5). Accordingly, hyperlipidemic conditions associated with elevated concentrations of total cholesterol and low density lipoprotein (LDL) cholesterol are significant risk factors.

Studies also show that a low plasma concentration of high density lipoprotein (HDL) cholesterol is a significant risk factor for the development of atherosclerosis (6) and that high levels are protective.

Lipoproteins are complexes of lipids and proteins held together by non-covalent bonds. Each type of lipoprotein class has a characteristic mass, chemical composition, density and physiological role. Irrespective of density or particle size, circulating lipids consist of a core of cholesteryl esters and triglycerides, and an envelope of phospholipids, free cholesterol and apolipoproteins. The apolipoproteins are involved in the assembly and secretion of the lipoprotein, provide structural integrity, activate lipoprotein-modifying enzymes, and are the ligand for a large assortment of receptors and membrane proteins.. Lipoprotein classes found in plasma include HDL, LDL, intermediate density lipoproteins (IDL) and very low density lipoproteins (VLDL).

Each type of lipoprotein has a characteristic apolipoprotein composition or ratio. The most prominent apolipoprotein in HDL is apolipoprotein-AI (apoA-I), which accounts for approximately 70% of the protein mass, with apoA-II accounting for another 20%. The ratio of apoA-I to apoA-II may determine HDL functional and anti-atherogenic properties. Circulating HDL particles consist of a heterogeneous mixture of discoidal and spherical particles with a mass of 200 to 400 kilo-daltons and a diameter of 7 to 10 nm.

HDL is one of the major classes of lipoproteins that function in the transport of lipids in plasma, and has multiple functions within the body, including reverse cholesterol transport, providing the cholesterol molecule substrate for bile acid synthesis, transport of clusterin, transport of paraoxanase, prevention of lipoprotein oxidation and selective uptake of cholesterol by adrenal cells. The major lipids associated with HDL include cholesterol, cholesteryl ester, triglycerides, phospholipids and fatty acids.

To better understand how HDL is anti-atherogenic, a brief explanation of the atherosclerotic process is necessary. The atherosclerotic process begins when LDL becomes trapped within the vascular wall. Oxidation of this LDL results in the binding of monocytes to the endothelial cells lining the vessel wall. These monocytes are activated and migrate into the endothelial space where they are transformed into macrophages, leading to further oxidation of the LDL. The oxidized LDL is taken up through the scavenger receptor on the macrophage, leading to the formation of foam cells. A fibrous cap is generated through the proliferation and migration of arterial smooth muscle cells, thus creating an atherosclerotic plaque.

HDL is essential for the transport of cholesterol from extra-hepatic tissues to the liver, where it is excreted into bile as free cholesterol or as bile acids that are formed from cholesterol. The process requires several steps. The first is the formation of nascent or pre-beta HDL particles in the liver and intestine. Excess cholesterol moves across cell membranes into the nascent HDL through the action of the ABCAI transporter. Lecithin cholesterol acyl transferase (LCAT) converts the cholesterol to cholesteryl ester and the subsequent conversion of nascent HDL to mature HDL. Esterified cholesterol is then transferred by cholesteryl ester transfer protein (CETP) from HDL to apolipoprotein-B containing lipoproteins, which are taken up by numerous receptors in the liver. Nascent HDL is regenerated via hepatic triglyceride lipase and phospholipid transfer protein and the cycle continues. In addition to the cholesterol removed from peripheral cells, HDL accepts cholesterol from LDL and erythrocyte membranes. Another mechanism of reverse cholesterol transport may involve passive diffusion of cholesterol between cholesterol-poor membranes and HDL or other acceptor molecules.

HDL protects against the development of atherosclerosis both through its role in reverse cholesterol transport and possibly by impeding LDL oxidation. Several HDL-associated enzymes are involved in the process. Paraoxonase (PON1), LCAT, and platelet activating factor acetylhydrolase (PAFAH) all participate by hydrolyzing phospholipid hydroperoxides generated during LDL oxidation and act in tandem to prevent the accumulation of oxidized lipid in LDL. These enzymes are responsible for the anti-oxidative and anti-inflammatory properties of HDL.

Although hypercholesterolemia is an important risk factor for patients with CVD, other risk factors such as

increased oxidative stress and serum homocysteine must be considered (7). Modification of these harmful components in the arteries would be beneficial in creating new avenues for management of those who have or are at risk of developing CVD but do not have hypercholesterolemia (8).

It is well documented that oxidative stress has an important role in the initiation and propagation of many chronic diseases, including atherosclerosis (9). Reactive oxygen species (ROS) are ubiquitous and occur naturally in all aerobic species, arising from both endogenous production of metabolism and as exogenous sources derived from environmental sources. ROS are largely indiscriminatively reactive molecules which readily damage biological macromolecules including DNA, protein, carbohydrates and lipids (10) Excessive or uncontrolled production of ROS has been implicated in the development of atherosclerosis and CVD at different stages, including vascular endothelial cell damage, foam cell formation, vascular smooth muscle proliferation, gene expression, impaired vasomotor reactivity and plaque instability (11,12a).

In the absence of adequate endogenous antioxidant defenses, the propagation of free radicals events can lead to the co-oxidation of nucleophilic cellular constituents as well as the reaction of secondary lipid autooxidation products with nucleophilic macromolecules. Examples of oxidative stress indicators in target tissues and blood include 8-hyroxydeoxyguanosine, malondialdehyde, 4-hydroxynoneanal and lipid peroxides, which include not only fatty acid oxidation products but also cholesterol oxidation products. Management of oxidative stress is achieved by the collective removal and detoxificiation of ROS from specific enzymatic and nonenzymatic cellular antioxidant systems. For example, enzymatic antioxidants involved in the detoxification of lipid and oxygen radicals include the Cu/Zn superoxide dismutase (SOD; cytoplasm), catalase (peroxisomes) selenium-glutathione peroxidatse/reductase redox cycle enzymes (GSH-Px and GSHG-Red in both the cytoplasm and mitochondria and finally the non-selenium glutathione-S-transferases (cytoplasm). Non enzymatic cellular antioxidants include $\alpha$-tocopherol, ascorbic acid and $\beta$-carotene.

Evidence from in vitro and in vivo studies suggests that oxidative modification of LDL is involved in the onset of atherosclerosis and exacerbates its clinical manifestations. Specific products of cholesterol oxidation have for example been identified in atheroscerotic plaque (12b). Accordingly, antioxidants especially those with dietary sources which can impact on either enhancing enzymatic antioxidant activity or complement non-enzymatic antioxidant activity have received considerable attention as possible agents that can protect against generation of ROS and thus the development of atherosclerosis and CVD.

The treatment of CVD with rice diets was suggested several decades ago. More than fifty years ago, it was reported that the consumption of white rice decreased blood pressure and lowered hypercholesterolemia in humans (13). However, there are many different types of rice. By far the most common rice consumed by human is white rice (over 85%), followed by red and black rice. The latter two are cultivated mainly in South Asia, Greece, Italy, and the United States. While Europeans eat more coloured rice than South Asians (14), coloured rice has longed been consumed in China and is considered to be a health food which confers body-strength. Thus, it is commonly known as a "blood strengthening rice" or "drug rice".

Black rice, which has a pigment level of 1 mg per 100 g rice, has 3 mg vitamin C and 0.2 mg riboflavin per 100 g and has more iron, calcium and phosphorus than non-pigmented rice. In Kerala, India, the variety Navara is believed to have medicinal properties and is used to rejuvenate the nerves in paralytic conditions: oridine, an alkaloid present in rice, has some antineurotic properties when impure.

Prior research in the field of black rice has focused on the preparation and use of black rice pigment as a colouring a gent (Chinese Patent No. 93109627.8), beverages and foods supplemented with black rice pigment (Chinese Patent No. 93109627.8) and the effects of whole dietary black and red rice on serum lipids and aortic plaque (15)

Su Noh Ryu et al: "High performance liquid chromatographic determination of anthocyanin pigments in some varieties of black rice", Journal of Food and Drug Analysis 6(4) 729-736 1998, discloses the isolation and extraction of cyanidin 3-glucoside and peonidin 3-glucoside from pigmented rice.

W.H. Ling et al: "Red and black rice decrease atheroslerotic plaque formation and increase antioxidant status in rabbits", J. Nutr., vol. 131, 2001, pages 1421-1426 discloses that black rice extract is known to improve the lipid profile and increase circulating HDL. >

It is an object of the present invention to obviate or mitigate the disadvantages and insufficiencies of compounds and compositions known in this field to treat and prevent CVD and all of its underlying conditions, including atherosclerosis, oxidative stress, and dyslipidemic conditions. It is a further object of the present invention to manipulate black rice so as to optimize its' various therapeutic effects.

## SUMMARY OF THE INVENTION

The present invention provides, in one aspect, a medical use of a composition comprising anthocyanins from black rice (Oryza sativa L) prepared by:

a) separating an outer layer from a starchy endosperm in de-hulled black rice;

b) adding a solution of at least one organic solvent and an acid to the separated outer layer;
c) filtering and removing the solvent and the acid from the separated outer layer to produce a pigment fraction;
d) separating constituents of the pigment fraction; and
e) collecting the anthocyanin composition therefrom.

The present invention comprises the use of a composition comprising cyanidin-3-O-glucoside and peonidin-3-O-glucoside in the manufacture of a medicament or nutraceutical for reducing LDL cholesterol.

The present invention further provides a method of treating or preventing cardiovascular disease and its underlying conditions, including atherosclerosis, inflammation, hyperlipidemic conditions, hypoalphalipoproteinemia, hypercholesterolemia, and oxidative stress in an animal which comprises administering a composition prepared by the extraction process of the present invention.

One of the key aspects of the process of the present invention is that anthocyanins, the active therapeutic components black rice, have been found to be most advantageously extracted from the separated outer layer of the rice granule. Distribution of anthocyanins is clearly related to the depth of the granule, with the first outer layer containing the highest level of anthocyanin. Prior known processes for the extraction of anthocyanins from black rice have not appreciated this important advantage.

These effects and other significant advantages are described in more detail below.

## BRIEF DESCRIPTION OF THE DRAWINGS

The present invention is illustrated by way the following non-limiting drawings in which:

Figure 1 is a diagram showing the components of a rice granule in cross-section;

Figure 2 is a chromatogram of Bio-Gel P-2 elution of black rice extract, with peaks 1 and 2 being subsequently identified by LC/MS as peonidin-3-glucoside and cyanidin-3-glucoside respectively;

Figure 3 is a chromatogram of Bio-Gel P-2 elution of black rice extract;

Figure 4 is a chromatogram of Bio-Gel P-2 elution of black rice extract, with a peak being subsequently identified by LC/MS as peonidin-3-glucoside;

Figure 5 is a chromatogram of Bio-Gel P-2 elution of black rice extract, with a peak being subsequently identified by LC/MS as cyanidin-3-glucoside;

Figure 6 is a graph showing the effect of black rice extract on inhibition of DPPH radical;

Figure 7 is a graph showing the effect of black rice extract on the formation of conjugated diene in peroxyl radical induced liposome peroxidation at 37° C;

Figure 8 shows the agarose gel electrophoresis results of the effect of black rice extract in suppressing the negative charge of oxidative modified LDL wherein lane 1= native LDL, lane 2=LDL plus cupric ion, lane 3-7=LDL plus cupric ion with black rice extract, and lane 8= LDL plus cupric ion and EDTA;

Figure 9 is a bar graph showing the suppression of LDL oxidative modification of human LDL *in vitro* (CD and TBARS);

Figure 10 shows the agarose gel electrophoresis results of the effect of black rice extract in preventing peroxyl radical induced super-coiled DNA scission wherein S= super-coiled DNA, lane 1 = D NA plus P BS, lane 2 =DNA plus AAPH, lane 3 , 4 , 5 a nd 6=DNA plus AAPH + 1,10,25, 100$\mu$g/ml black rice extract, respectively, lane 7,8= DNA plus AAPH + 1,10$\mu$g/ml Trolox™, respectively;

Figure 11 shows the agarose gel electrophoresis results of the effect of the combination of peonidin-3-glucoside and cyanidin-3-glucoside (10$\mu$g/ml) in reducing the damage of peroxyl radical induced DNA scission wherein lane 1,2= native and oxidative DNA respectively, lanes 3, 4, 5, 6 and 7 =DNA plus AAPH with 9/1, 4/1, 1/1, 1/4 and 1/9 of cyanidin-3-glucoside and peonidin-3-glucoside respectively, lane 8= DNA plus AAPH with 10$\mu$g/ml Trolox™;

Figure 12 shows the agarose gel electrophoresis results of the effect of black rice extract in preventing hydroxyl radical (non-site specific) induced DNA nicking wherein lane 1= DNA plus PBS, lane 2=DNA plus hydroxyl radical

initiator, lanes 3 and 4=DNA plus hydroxyl radical initiator + 1.7, 17 mg/ml black rice extract (first layer), respectively, lanes 5,6= DNA plus hydroxyl radical initiator + 1.7, 170mg/ml extract of whole granule of black rice, respectively, lanes 7,8= DNA plus hydroxyl radical initiator +0.17, 1.7 mg/ml Trolox™ respectively;

Figure 13 shows the agarose gel electrophoresis results of the effect of black rice extract in preventing hydroxyl radical (site specific) induced DNA nicking wherein lane 1= DNA plus PBS, lane 2=DNA plus hydroxyl radical initiator, lanes 3 and 4=DNA plus hydroxyl radical initiator + 1.7, 17 mg/ml black rice extract (first layer), respectively, lanes 5,6= DNA plus hydroxyl radical initiator + 1.7, 170mg/ml extract of whole granule of black rice, respectively, lanes 7,8= DNA plus hydroxyl radical initiator +0.17, 1.7 mg/ml Trolox™ respectively;

Figure 14 is a bar graph showing cell viability test using black rice extract;

Figure 15 is a bar graph showing inhibition of hepatic lipase activity by black rice extracts.

Figure 16 is a graph representing the body weights of mice in different treatment groups over time;

Figure 17 is a graph representing the areas of atherosclerotic plaque in different groups. The extent of atherosclerosis was quantified after 16 wk. Atherosclerotic lesion area in the sinus was measures by staining lesions with oil-red O. Values are means $\pm$ SD, n=15.Bars without common letters are significantly different, P<0.01;

Figure 18 is a graph showing effect of the black rice fraction of the present invention on the inhibition of nitric oxide stimulated by bacterial lipopolysacharide in mouse macarophage cell RAW264.7; and

Figure 19 shows the agarose gel electrophoresis results of the effect of the black rice extract of the present invention on the expression of inducible nitric oxide synthase.

## PREFERRED EMBODIMENTS OF THE INVENTION

The following detailed description is provided to aid those skilled in the art in practising the present invention. However, this detailed description should not be construed so as to unduly limit the scope of the present invention. Modifications and variations to the embodiments discussed herein may be made by those with ordinary skill in the art without departing from the scope of the present invention.

### Extraction Process

According to the present invention, there is provided a process of extracting a composition comprising anthocyanins from black rice (Oryza sativa L) which comprises separating an outer layer from a starchy endosperm in de-hulled black rice; adding a solution of at least one organic solvent and an acid to the separated outer layer; filtering and removing the solvent and the acid from the separated outer layer to produce a pigment fraction; separating constituents of the pigment fraction; and collecting the anthocyanin composition therefrom.
The starting material for the process of the present invention is de-hulled black rice. Dehulling of the rough rice may be carried out by a variety of ways known in the art. Generally, hulling is done either manually (hand pounding) or mechanically. Mechanical hullers are of three main types: Engelberg mills, stone dehullers and rubber dehullers. Stone dehullers are still common in tropical Asia. Rubber rollers are common in Japan, where de-hulled rice is stored instead of rough rice, with a resultant space saving.
The first step of the process of the present invention is critical and comprises separating the outer layer from the starchy endosperm in de-hulled black rice. For greater understanding, Figure 1 shows the basic components of a rice granule in cross-section. After removal of the husk components, for example cellulose and hemi-cellulose in the de-hulling process, what generally remains are the pericarp, tegmen and aleurone layers which are positioned over the starchy endosperm. Within the scope of the present invention, it is desired to separate this outer "aleurone" layer and from this, extract an anthocyanin composition. Heretofore, the entire rice granule has been used in anthocyanin extraction processes but never just this outer layer.
It is preferred that the separation of the outer layer from the starch endosperm be achieved by physical separation, most preferably by milling. Various types of milling are already used in the manufacture and refinement of coloured rice and may be employed herein. In such prior known manufacture, the outer layers are generally discarded as it is the de-hulled, milled and then polished rice granules which are purchased by consumers. Within the scope of the present invention, it is the milled material which is retrieved and from which the anthocyanin composition is ultimately extracted.
There are many commonly used rice mills from a single-pass Engelberg mill to multipass systems. Within the

scope of the present invention, manual technology involving hand pounding may be used as well as machine-milling using abrasion or friction. Most preferably, a mill using physical scratching is employed. Examples of suitable mills are provided by Satake USA Inc. and Buhler AG.

Slender grains require less pressure to mill than bold (i.e. thick) grains because of their thinner aleurone layer. Milling is undertaken until substantially all of the outer layer (comprising the aleurone layer) is removed. Generally, this layer comprises 5 to 15% of the rice granule. In one preferred form, as an index to determine the end of the milling process, one may gauge approximately 10% of the rice granule. Alternatively, one may assess the granule colour change and end the milling process as the granule becomes light coloured relative to the starting material. Processing can be adjusted for the required depth of granule.

Innovations introduced in the Japanese rice industry which may be taken advantage of within the scope of the present invention include microcomputer control of milling based on the desired degree of milling and germ rice milling. A germ rice milling machine introduced in 1976 that uses gentle, abrasive roll milling under very low pressure leaves the germ intact for more than 80 percent of the grains. This way, greater economic efficiency is achieved as the outer layer is used to extract therapeutically useful anthocyanin compositions and the remainder of the granule may be sold as a commercial product.

Table I shows the proximate analysis of whole black rice and milled black rice outer layer fraction from a Shimzu Mill for comparison:

| | Black Rice (outer layer) | Black Rice (whole granule) |
|---|---|---|
| Protein g/100mg | 17 | 10.0 |
| Fat g/100mg | 9.8 | 2.0 |
| Moisture g/100mg | 8.3 | 11.0 |
| Ash g/100mg | 7.6 | 1.4 |
| Carbohydrate g/100mg | 57.0 | 76.0 |
| Energy kjoules/100mg | 1610 | 1510 |

Clearly, the outer layer fraction has a higher concentration of nutrients per 100mg than the whole granule.

Table 2 shows the comparative distribution of anthocyanin in black rice, both whole granule and fractions thereof

| Fraction | Mg/kg |
|---|---|
| Whole granule | 1645±25 |
| 1st layer | 13,567±802 |
| 2nd layer | 3850±876 |
| Inside kernel | 70±9 |

It has been found that the distribution of anthocyanin (combination of both cyanidin-3-glucoside and peonidin-3-glucoside) is related to the depth of the granule, with the first layer separated from the starchy endosperm containing the highest amount of anthocyanin. This finding provides a unique opportunity to optimize anthocyanin recovery.

The second step of the process of the present invention comprises adding a solution of at least one organic solvent and an acid to the separated outer layer. Preferably, the organic solvent is selected from the group consisting of alcohols, ketones, hydrocarbons and water.

The ketones are selected from the group having the general structure $RCOR^1$ where R and $R^1$ are alkyl groups. Preferably the alkyl groups are $C_1$ - $C_6$ groups. Most preferably, the ketone is 2-propanone (acetone). The hydrocarbon may be selected from the group comprising all $C_5$ - $C_{10}$ hydocarbons. Most preferably, the hydrocarbon is hexane. The alcohol is selected from the group having the general structures R-CHOHR, R-$CH_2$OH, and RCOH where R is a $C_1$ - $C_4$ alkyl group. Most preferably, the alcohol is either methanol or ethanol.

The acid may be any non-toxic, edible acid sufficient to attain the desired pH range. Preferably, the acid is selected from the group consisting of hydrochloric acid, acetic acid, citric acid, tartaric acid and low concentration sulphuric acid. It is preferred that sufficient acid is added to achieve a pH of between 1 and 4. It is preferred that the separated outer layer is soaked in the solvent for a period of between 1 and 10 hours and that the temperature be in the range of from 25-40° C. It is preferred that the ratio of solvent to rice be between 30/1 to 100/1 (volume/weight).

The third step of the process of the present invention comprises filtering and removing the solvent and the acid from the separated outer layer to produce a pigment fraction. Preferably, the solvent is removed by filtrate extraction and the acid removed by evaporation, such as by rotary evaporation although other methods are clearly within the purview of the present invention, for example, vacuum drying and freeze drying.

The fourth and fifth steps of the process of the present invention comprise separating and collecting the constituents of the pigment fraction. Preferably this is achieved via fractionation. Most preferably, procedures such as column chromatography, (for example, polyamide gel filtration) may be used. At this stage, after fractionation, two major fractions are collected, one comprising cyanidin-3-glucoside and the other comprising peonidin-3-glucoside.

Verification of the identity of the fractions after separation may be achieved by high pressure liquid chromatography (HPLC), liquid chromatography/mass spectrometry (LC/MS) as required. Figure 2 is a chromatogram of Bio-Gel P-2 elution of black rice extract, with peaks 1 and 2 being subsequently identified by LC/MS as peonidin-3-glucoside and cyanidin-3-glucoside respectively.

Preferred black rice varieties, which may be used in accordance with the present invention include, but are not limited to: Wu Gong, Shang-nong, Hei Xiang Geng Nuo, Zhen Xi Hei Mi, Dong Bei Nuo 149, Ao Yu Nuo 349, Hong 280, Hong 463 and Xiang Xue Nuo.

**Anthocyanin Compositions**

The present invention comprises compositions which comprise cyanidin-3-O-glucoside and peonidin-3-O-glucoside derived from black rice. Such compositions include those compositions derived from the extraction and purification process described herein and also those compositions which are synthesized de novo (i.e. not necessarily derived from this process) having the ratios of the two constituents as described further hereinbelow.

The structures of cyanidin-3-O-glucoside and peonidin-3-O-glucoside are as follows:

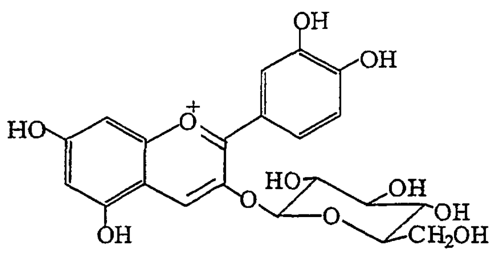

cyanidin-3-glucoside

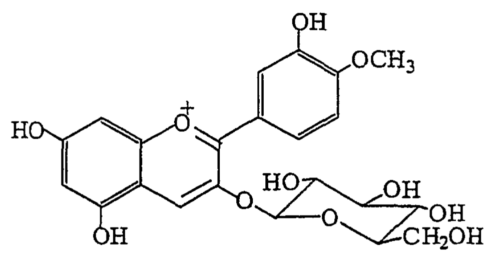

peonidin-3-glucoside

In a preferred embodiment, the composition of the present invention comprises cyanidin-3-glucoside and peonidin-3-glucoside in a ratio of from 10 to 1, more preferably from 6 to 1, more preferably from 3.5:1 to 6.5:1. Most preferably, this ratio is 4:1.

Within the scope of the present invention, cyanidin-3-glucoside and peonidin-3-glucoside, for use in preparing such compositions, may be extracted or obtained from a number of sources, including but not limited to black rice, black fruit sources such as blackberries and black currant, black beans, black seeds such as sesame, and black grains such as black wheat. In addition, other sources include red currant and red rice.

In one embodiment of the present invention, in order to achieve compositions having the preferred anthocyanin ratios as described above, a most preferred process to "blend" anthocyanins from varying sources. This way, one may take advantage of particularly high levels of one component from one while another source may be richer in the other required component.

<u>Combination with Anti-Oxidants and/or Phytosterols</u>

In a further embodiment, the compositions of the present invention may be combined, prior to administration, co-adminstered or administered separately over a time interval with one or more anti-oxidants. Suitable anti-oxidants include, but are not limited to: vitamin E, beta-carotene, enzymatic superoxide dismutase, catalase, glutathion peroxidase, glutathione reductase, tea catechins, chelating agents such as citric acid, EDTA, phenylalanine, phosphoric acid, tartaric acid and tryptophane; preferentially oxidized compounds such as ascorbic acid, sodium bisulfite and s odium sulfite;

water soluble chain terminators such as thiols and lipid soluble chain terminators such as alkly gallates, ascorbyl palmitate, t-butyl hydroquinone, butylated hydroxyanisole, butylated hydroxytoluene, hydroquinone, nordihydroguaiaretic acid and alpha-tocopherol. It is believed that the combination of these anti-oxidants and the anthocyanin compositions of the present invention initiates and perpetuates the beneficial anti-oxidant effects.

In a further embodiment, the compositions of the present invention may be combined, prior to administration, co-adminstered or administered separately over a time interval with one or more sterols or stanols.

As used herein, the term "sterol" includes all sterols without limitation, for example: sitosterol, campesterol, stigmasterol, brassicasterol (including dihydrobrassicasterol), desmosterol, chalinosterol, poriferasterol, clionasterol, ergosterol, coprosterol, codisterol, isofucosterol, fucosterol, clerosterol, nervisterol, lathosterol, stellasterol, spinasterol, chondrillasterol, peposterol, avenasterol, isoavenasterol, fecosterol, pollinastasterol, cholesterol and all natural or synthesized forms and derivatives thereof, including isomers. The term "stanol" refers to saturated or hydrogenated sterols including all natural or synthesized forms and derivatives thereof, and isomers. It is to be understood that modifications to the sterols and stanols i.e. to include side chains also falls within the purview of this invention. For example, the purview of this invention clearly includes 24 beta-ethylchlostanol, 24-alpha-ethyl-22-dehydrocholstanol. It is also to be understood that, when in doubt throughout the specification, and unless otherwise specified, the term "sterol" encompasses both sterol and stanol. In a most preferred form, the sterol is in its saturated form and is sitostanol.

These sterols and stanols for use in accordance with this invention may be procured from a variety of natural sources. For example, they may be obtained from the processing of plant oils (including aquatic plants) such as corn oil and other vegetable oils, wheat germ oil, soy extract, rice extract, rice bran, rapeseed oil, sunflower oil, sesame oil and fish (and other marine-source) oils. They may also be derived from fungi, for example ergosterol, or animals, for example cholesterol. Accordingly, the present invention is not to be limited to any one source of sterols. US Patent Serial No. 4,420,427 teaches the preparation of sterols from vegetable oil sludge using solvents such as methanol. Alternatively, phytosterols and phytostanols may be obtained from tall oil pitch or soap, by-products of forestry practises as described in US Patent Serial No.5,770,749, incorporated herein by reference.

It is believed that the combination of these sterols and/or stanols in conjunction with the anthocyanin compositions of the present invention yields at least additive and perhaps synergistic and complementary therapeutic effects, particularly in lipid modulation. While not intending to be bound by any one theory regarding the mechanism of action, it is possibly due to the different lipid targets of each component. For example, sterols and stanols have been shown to be effective agents to lower serum LDL-C while they show minimal efficacy in increasing serum HDL-C. Conversely, the anthocyanin and sterol/stanol compositons of the present invention have been found to effectively and quite substantially to stabilize both HDL-C and LDL-C from oxidation. In addition, there is a complementary effect between the anthocyanin components and the sterol/stanol components on the reduction and substantial arrest of atherosclerotic lesion development.

## Therapeutic Efficacy

The composition comprising cyanidin-3-O-glucoside and peonidin-3-O-glucoside can be used for reducing LDL cholesterol. This medical use is related with a method of treating or preventing cardiovascular disease and its underlying conditions, including atherosclerosis, inflammation, hyperlipidemic conditions, hypoalphalipoproteinemia, hypercholesterolemia, and oxidative stress in an animal, preferably a human, which comprises administering to the animal a therapeutically effective amount of a composition derived from black rice which comprises cyanidin-3-glucoside and peonidin-3-glucoside and optionally one or more phytosterols or phytostanols and/or one or more antioxidants.

Such compositions include those in which:

1) the anthocyanin components are derived from the extraction and purification process described herein;
2) the anthocyanin components are synthesized de novo and having the ratios of the two anthocyanin constituents as described herein; and
3) the anthocyanin components are extracted and purified from more than one source, for example, black rice, black bean, black seeds, blackberries, and the blended to achieve the desired ratio cyanidin-3-glucoside and peonidin-3-glucoside.

The term "therapeutically effective" is intended to qualify the amount of the composition administered in order to achieve one or more of the following goals:

a) enhancing and/or preserving the stability of HDL from oxidation;
b) enhancing and/or preserving the stability of LDL, VLDL or IDL from oxidation
c) enhancing and/or preserving the stability of triglyceride (TG) from oxidation;
d) preventing, reducing, eliminating or ameliorating a dyslipidemic condition or disorder;

e) preventing, reducing, eliminating or ameliorating hypercholesterolemia, hypoalphalipoproteinemia,

f) preventing, reducing, eliminating or ameliorating the development of atherosclerotic lesions;

g) preventing, reducing, eliminating or ameliorating the development of inflammation associated with the development of cardiovascular disease and coronary artery disese;

h) preventing, reducing, eliminating or ameliorating any condition, disease or disorder which has as its basis or which is exacerbated by a deficiency in plasma HDL, or excess of either LDL, VLDL, Lp(a), beta-VLDL, IDL or remnant lipoproteins; and

i) preventing, reducing, eliminating or ameliorating injuries due to oxidative stress.

As will become apparent from the examples provided below, the compositions of present invention exhibit strong anti-oxidant effects as evidenced by numerous testing protocols and are effective at decreasing atherosclerotic plaque formation.

It has been found that the anthocyanin compositions of present invention, when administered therapeutically:

1) enhances and/or preserves the stability of HDL-C and the atherogenic lipioproteins such as LDL-C, VLDL-C, and IDL-C from oxidation;

2) prevents, reduces, eliminates or ameliorates injuries due to oxidative stress;

3) prevents, reduces, eliminates or ameliorates the development of atherosclerotic lesions; and

4) prevents, reduces, eliminates or ameliorates inflammation associated with atherosclerosis, coronary artery disease (CAD), and cardiovascular disease (CVD). This is critical as it has recently been found that CAD and CVD occur in apparently low-risk people due to inflammation. Recent evidence also suggests that inflammation of the arteries may be an important indicator of future heart attacks and strokes. Inflammation occurs when the body responds to injury or infection.

It has been found that the anthocyanin and phytosterol/phytostanol compositions of present invention, when administered therapeutically:

1) prevents, reduces, eliminates or ameliorates the development of atherosclerotic lesions;

2) modulates or controls plasma lipoproteins;

3) provides for the prevention, reduction, elimination or amelioration of a number of conditions and disorders, including, but not limited to: cardiovascular disease and its underlying conditions, including atherosclerosis, dyslipidemic conditions or disorders, hypercholesterolemia, and hypoalphalipoproteinemia, development of atherosclerotic lesions and toxic shock syndrome; and

4) provides for the prevention, reduction, elimination or amelioration of a number of conditions and disorders which have as their basis or which are exacerbated by a deficiency in plasma HDL, or excess of either LDL, VLDL, or IDL.

**Methods of Use**

The compositions of the present invention may be administered by any conventional means available for use in conjunction with pharmaceuticals, nutraceuticals, foods, beverages, and the like.

Without limiting the generality of the foregoing, the compositions of the present invention may be admixed with various carriers or adjuvants to assist in direct administration or to assist in the incorporation of the composition into foods, beverages, nutraceuticals or pharmaceuticals. In order to appreciate the various possible vehicles of the delivery of the compositions, the list below is provided. The doses of the composition will vary depending upon, among other factors, the mode of delivery, the patient size and condition, the result to be achieved, as well as other factors known to those skilled in the art of food additives and medicinal agents.

The desired effects d escribed herein may be achieved in a number of different ways. These compositions may be administered by any conventional means available for use in conjunction with pharmaceuticals, nutraceuticals, foods, beverages, and the like.

The amount of the composition which is required to achieve the desired effects will, of course, depend on a number of factors such as the particular profile of the composition, the mode of administration and the condition of the patient.

<u>1) Pharmaceutical Dotage Forms:</u>

The compositions of the present invention can be administered to a patient either by themselves, or in pharmaceutical compositions where they are mixed with suitable carriers or excipients.

Use of pharmaceutically acceptable carriers to formulate the compositions herein disclosed for the practice of

the invention into dosages suitable for systemic administration is within the scope of the invention. With proper choice of carrier and suitable manufacturing practice, the compositions of the present invention, in particular, those formulated as solutions, may be administered parenterally, such as by intravenous injection. The compositions can be formulated readily using pharmaceutically acceptable carriers well known in the art into dosages suitable for oral administration. Such carriers enable the compositions of the invention to be formulated as tablets, pills, capsules, liquids, gels, syrups, slurries, suspensions and the like, for oral ingestion by a patient to be treated.

Pharmaceutical formulations, comprising one or more of the compositions of the present invention, include formulations wherein the active ingredients are contained in an effective amount to achieve their intended purpose. Determination of the effective amounts is well within the capability of those skilled in the art, especially in light of the detailed disclosure provided herein.

In addition to the active ingredients these pharmaceutical compositions may contain suitable pharmaceutically acceptable carriers comprising excipients and auxiliaries which facilitate processing of the active compounds into preparations which can be used pharmaceutically. The preparations formulated for oral administration may be in the form of tablets, dragees, capsules, or solutions.

The pharmaceutical compositions of the present invention may be manufactured in a manner that is itself known, e.g., by means of conventional mixing, dissolving, granulating, dragee-making, levigating, emulsifying, encapsulating, entrapping or lyophilizing processes.

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

Pharmaceutical preparations for oral use can be obtained by combining the active compounds with solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate.

Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added.

Oral liquid preparations may be in the form of, for example, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia; nonaqueous vehicles (which may include edible oils), for example almond oil, fractionated coconut oil, oily esters such as esters of glycerine, propylene glycol, or ethyl alcohol; preservatives, for example methyl or propyl p-hydroxybenzoate or sorbic acid; and if desired conventional flavouring or colouring agents.

It is contemplated within the scope of the present invention that the compositions of the present invention may be incorporated into various conventional pharmaceutical preparations and dosage forms such as tablets (plain and coated) for use orally, bucally or lingually, capsules (hard and soft, gelatin, with or without additional coatings) powders, granules (including effervescent granules), pellets, microparticulates, solutions (such as micellar, syrups, elixirs and drops), lozenges, pastilles, ampoules, emulsions, microemulsions, ointments, creams, suppositories, gels, transdermal patches and modified release d osage forms together with customary excipients and/or diluents and stabilizers.

The compositions of the present invention, adapted into the appropriate dosage form as described above may be administered to animals, including humans, orally, by injection (intravenously, subcutaneously, intra-peritoneally, intra-dermally or intra-muscularly), topically or in other ways.

The precise modes of delivery of the composition of the present invention in each case will depend upon the objectives of the administration protocol. In the case of existing conditions and disorders, it will depend upon the severity

of the disorder, and as discussed above, the age, size and gender of the individual. Determining appropriate dosages and administration schedules is well within the purview of one skilled in this field.

2) Foods/Beverages/Nutraceuticals:

In another form of the present invention, the compositions of the present invention may be incorporated into foods, beverages and nutraceuticals, for on-going prophylactic use, including, without limitation, into the following:

1) Dairy Products --such as cheeses, milk and other dairy beverages, spreads and dairy mixes, ice cream and yoghurt;
2) Cereal-Based Products--comprising grains (for example, bread, pastas, crackers and cereal bars) whether these goods are cooked, baked or otherwise processed;
3) Confectioneries-such as chocolate, candies, chewing gum, desserts, non-dairy toppings (for example Cool Whip™), sorbets, icings and other fillings;
4) Beverages- whether alcoholic or non-alcoholic and including colas and other soft drinks, juice drinks, dietary supplement and meal replacement drinks such as those sold under the trade-marks Boost™ and Ensure™; and
5) Miscellaneous Products--including processed foods such as soups, pre-prepared pasta sauces, pre-formed meals and the like.

The compositions of the present invention may be incorporated directly and without further modification into the food, nutraceutical or beverage by techniques such as mixing, infusion, injection, blending, dispersing, emulsifying, immersion, spraying and kneading. Alternatively, the compositions may be applied directly onto a food or into a beverage by the consumer prior to ingestion. These are simple and economical modes of delivery.

**EXAMPLES**

The present invention is described by the following non-limiting examples:

**Example 1 Extraction and Separation of Anthocyanin**

The outer layer was removed from Wu Gong black rice by Shimzu Mill in POS (Saskatchewan). Black rice fraction was soaked in 1% HCl in methanol overnight. Whatman filter paper no 4 was u sed to filtrate extraction. M ethanol was removed b y rotary evaporation under 40° C. Obtained pigment fraction was loaded into Bio-Gel P-2 column (2.5 x 45cm) packed with water acidified to pH 2.5 with acetic acid to separate anthocyanin.
The column was eluted with water acidified to pH 2.5 with acetic acid at a flow rate of 1ml/min and fractions were collected by fractionation collector (5ml/tube or 5 min/tube). The sample was monitored at 520nm. Two major fractions were collected and concentrated under vacuum for identification.

**Example 2 Quantitation and Identification of Anthocyanin**

The two major fractions from example 1 were further analyzed by HPLC and LC/MS using standard reference (cyanidin-3-glucoside and peonidin-3-glucoside). The HPLC was performed using a Waters Alliance 2690 system equipped with an autosampler and column heater. HPLC conditions were as follows: Waters Xterra MS C18 column (2.1x50mm, 2.5μm) was used at 40°C), 5μl injection, solvent A was 100% methanol and solvent B was 5% fornic acid in water. Concentration of A was 20-4-% within 5 minutes. Flow rate was set as 0.4ml/min. Waters996 PDA detector was used and wavelength set at 200-600nm.
LC/MS analysis of black rice fractions was conducted using electrospray Mass spectrometry coupled with Waters 2690 separation module. PDA showed that using this Bio-Gel P-2 chromatography, two fragments were obtained which were distinguishable in both retention time and spectrum (Figures 3, 4 and 5). Figure 3 shows fraction I and fraction II after Bio-Gel P-2 separation.

**Example 3 Anti-Oxidant Activity DPPH Radical Scavenging Test**

The radical scavenging activity of the first layer, second layer and whole granule of black rice was evaluated using a stable free radical 1,1-diphenyl-2-picryl ("DPPH") in ethanol solution. DPPH will change into non-radical form when it reacts with other free radical scavengers, especially, primary anti-oxidants (hydrogen donors). A spectrometric

method was applied to measure the disappearance of DPPH in an alcohol solution.

Figure 6 shows the effect of each layer and the whole granule on the inhibition of the DPPH radical. Clearly, at each concentration tested, the first layer is superior.

**Example 4 Anti-Oxidant Activity Liposome Model**

The free radical scavenging activity of the anthocyanin compositon of the present invention was evaluated in a liposome model, where oxidation of a phospholipid was induced by thermolysis of 2,2-azobis(∝-amidinopropane) dihydrochloride ("AAPH"). This test was done by continuously monitoring the formation of conjugated diene hydroperoxide at 37°C.

Figure 7 shows the efficacy of the anthocyanin composition in preventing the formation of the conjugated diene hydroperoxide at various concentrations, with over 10μg/ml being most effective.

**Example 5 Suppression of LDL Oxidation**

The prevention of LDL oxidation by the anthocyanin composition of the present invention was evaluated. Oxidative modification of LDL is a critical step in the development of atherosclerosis due to the modification of down-regulation of ox-LDL. The modification of LDL particles results in the formation of foaming cells with the macrophage cell, and in turn the formation of laden and plaque on the endothelial of blood vessels. Accordingly, the reduction of LDL is considered as an important step in the prevention of cardiovascular disease. Transitional metal ion was removed by dialysis and LDL oxidation was initiated by co-incubating with the $Cu^{2+}$ ion at 37° C for a period of time.. LDL oxidation was monitored by different approaches, including agarose gel electrophoresis, formatiom of conjugated diene and thiobarbituric acid reactive substrate. The results in Figure 8 show that the addition of the anthocyanin composition of the present invention reduced the electrophoretic migration distance, indicating enhancement of negative charge of oxidatively modified LDL was suppressed.

Moreover, the formation of conjugated diene and thiobarbituric acid reactive substrates were also reduced (Figure 9), suggesting that the generation of primary and secondary lipid peroxidation products were also inhibited by the composition of the anthocyanin composition. In addition, HDL oxidation was evaluated. Peroxyl radical induced HDL modification was prevented by the addition of the anthocyanin composition as measured by agarose gel electrophoresis.

**Example 7 Suppression of DNA Oxidation**

The effects of the anthocyanin composition in preventing DNA oxidation induced by oxidative radicals such as the peroxyl radical and hydroxyl radicals (site specific and non-site specific) were investigated. Figure 10 shows that the peroxyl radical led to the disappearance of the supercoiled DNA strand and the addition of either the anthocyanin composition or the standard anti-oxidant Trolox resulted in partial recovery of such damage. A similar effect was seen by the addition of cyanidin-3-glucoside and peonidin-3-glucoside in combination at various ratios Figure 11).

The black rice extract or composition of the present invention inhibited the DNA scission-induced by both non-site specific and site-specific hydroxyl radical (Figures 12 and 13). In this hydroxyl radical model, the hydroxyl radical was generated by ascorbic acid mediated Fenton reaction. The black rice extract was found to scavenge the hydroxyl radical generated in the site-specific model and was also demonstrated to chelate transitional metal ion in the site-specific hydroxyl radical model.

**Example 8 Cell Viability Test**

The purpose of this test was to determine whether the extract of the present invention provided protection a gainst free-radical induced cytotoxicity in cell culture. In THP-1, a leukemia human cell line (ATCC), the addition of ferrous ion (as oxidative stimulus) resulted in c ell d eath via oxidative stress attributed to the Fenton reaction mechanism (Figure 14). The addition of the black rice extract of the present invention recovered the cell death in a concentration dependent manner.

**Example 9 Hepatic Lipase Suppression**

Hepatic liapse activity was suppressed by the extract of the present invention (referred to as 1st layer in Figure 15). Hepatic lipase hydrolyzes HDL phospholipid and triglyceride, an dposttheparin plasma hepatic lipase activity is inversely related to plasm HDL-C levels. Accordingly, the results show that the extract of the present invention, high in anthocyanins, would likely increase HDL *in vivo.*

**Example 10 DNA nicking prevention**

Both peroxyl radical and hydroxyl radical were applied in this test to evaluate the effect of black rice extract of the present invention in preventing DNA scission by 0.7% agarose gel electrophoresis with 0.5μg/ml ethidium bromide using TAE buffer (40mM Tris-acetate, 2mM EDTA, pH8.5). DNA bands were visualized by ultraviolet bench top transilluminator (UVP Inc., Upland, CA) and densities of bands were analyzed by LabWork software (UVP Inc., Upland, CA). The inhibition of DNA nicking was calculated as following equation:

$$\%Inh = \frac{D_{native} - D_{sample}}{D_{native} - D_{oxidative}} \times 100$$

$D_{native}$, $D_{oxidative}$. $D_{sample}$ represented the density of DNA without AAPH, DNA with AAPH and DNA with AAPH and test sample, respectively.

DNA integrity is vital to cell division and survival. DNA has been shown to be vulnerable to oxidative damage *in vivo,* leading to disrupt transcription, translation and DNA replication and eventually mutation and cell death. For example, Schneider *et al.* [19] have demonstrated that hydroxyl radical implicated in DNA damage, suggesting that early hydroxyl radical is biologically associated with the mutagenic and carcinogenic processes. Transitional metal-ascorbic acid-hydrogen peroxide system produced sequence-dependent damage of DNA with preferential oxidation of guanines, suggesting that localized production of the reactive oxygen species as a result of metal binding to the special regions of the DNA. Previous reports indicated that DNA nicking test could be a useful tool for evaluating antioxidant activity against active oxygen species induced DNA deterioration including both hydroxyl radicals and peroxyl radicals [20](Kitts et al., 1999).

In the present study, we adapted the concept of both non-site specific and site-specific hydroxyl radical and applied these to the DNA scission assays (Figures 10 and 11). It is evident that hydroxyl radical, no matter site-specific or non-site specific broke the integrity of supercoiled DNA strand (lane 2 of Figure 10).

In this hydroxyl radical-induced DNA nicking test black rice extract showed higher affinity of prevention in the non-site specific case than that in the site-specific case (Table 3).

**Table 3.** Inhibition percentage of blackrice extract on the prevention of DNA nicking induced by hydroxyl radical

| μg/ml | | Anthocyanin content (%)[1] | Non-site specific | Site specific |
|---|---|---|---|---|
| Whole rice | | 1.36±0.01 | | |
| | 100 | | 65.7 | 51.4 |
| | 1000 | | 83.9 | 74.1 |
| Fraction | | 0.16±0.02 | | |
| | 10 | | 54.4 | 49.0 |
| | 100 | | 82.8 | 69.5 |
| Trolox | | | | |
| | 0.17 | | 44.0 | 25.7 |
| | 1.7 | | 80.8 | 61.0 |

[1] measured by LC-MS using cyanidin-3-glucoside and peonidin-3-glucoside as external standards.

It is also noteworthy that the prevention of supercoiled DNA from hydroxyl radical induced nicking was also associated with the anthocyanin distribution in the black rice, i.e., blackrice outer layer contains higher amount of anthocyanin exhibits higher capacity than whole rice in inhibiting hydroxyl radical induced DNA damage (Table 3). Standard antioxidant Trolox also showed similar trend as higher inhibition of DNA damage induced by non-site specific hydroxyl radical (Table 3), confirming that sequence-specification was involved in the hydroxyl radical induced DNA strand breakage. The relevance of hydroxyl radical induced DNA nicking is that Fenton reaction components, such as hydrogen peroxide and transitional metal ion, could be found in cell nucleus and in turn inducing cell toxicity associated with DNA scission.

**Table 4**. Effects of blackrice and anthocyanin in preventing supercoiled DNA strand against peroxyl radical induced scission

| Sample | | %Inh |
|---|---|---|
| Blackrice fraction, | 1μg/ml | 1.0±0.7 |
| | 10μg/ml | 7.4±0.3 |
| | 25μg/ml | 25.8±8.7 |
| | 100μg/ml | 82.2±7.8 |
| Cyanidin-3-glucoside:peonidin-3-glucoside | | 63.2±1.6 |
| | 9:1 | |
| | 4:1 | 50.4±2.1 |
| | 1:1 | 38.7±0.3 |
| | 1:4 | 50.2±0.6 |
| | 9:1 | 40.7±0.0 |
| Trolox, | 10μg/ml | 100 |

In our present study, we also found that the black rice extract suppressed the supercoiled DNA scission at a concentration dependent manner (Table 4), of which the significant effect was as low as 10μg/ml. As pointed above, cyanidin-3-glucoside and peonidin-3-glucoside exist in the black rice as major pigments contributors. In our study, we found that cyanidin-3-glucoside and peonidin-3-glucoside exhibited 11.5 and 11.4% protection on the DNA strand scission at 1μg/ml respectively, whereas, protections increased to 45.3% and 44.1% at 5μg/ml level, respectively. The effect of combination of cyanidin-3-glucoside and peonidin-3-glucoside was also measured in this peroxyl radical induced DNA scission model. Results showed that the protection decreased with the decrease of percentage of cyanidin-3-glucoside (Figure 10 and Table 3). A concentration-dependent suppression of supercoiled DNA scission was also observed with black rice extract (Figure 11 and Table 4).

**Example 11 Anti-Inflammation Testing**

**A) Effect of black rice extract on preventing nitric oxide**

**B) Western blotting for the iNOS**

**A)**

Mouse macrophage cell RAW 264.7 (ATCC) was cultured in DMEM medium supplemented with 10% fetal bovine serum and antibiotics (100U/ml of penicillin and 100U/ml of streptomycin) at 37°C under 5% $CO_2$, Cell was plated at a density of $2\times10^5$ cells/well into 96-well plates. After overnight growth when cells adhered to the bottom of well, various amount of the black rice extract in PBS and 1μg/ml bacterial lipopolysaccharide (LPS, *Escheridchia coli, serotype* 0111:B4) were added for another 24 hr. Medium was aliquot (100μl) to another 96-well plate where 100μl of Greiss reagent (1% sulfanilamide in 5% phosphoric acid and 0.1 % naphthylethylenediamine dihydrocholide in water, 1:1 v/v) was then added [18]. Absorbance at 540nm was determined with ELISA plate reader. The concentration of nitrite was measured in according to standard curve obtained from the same procedure. The inhibition of nitric oxide was calculated according to the following equation:

$$\%Inhibtion = \frac{Abs_{positive} - Abs_{sample}}{Abs_{positive} - Abs_{negative}} \times 100$$

of which, $Abs_{positive}$, $Abs_{negative}$, $Abs_{sample}$ represent absorbance of cultural media with LPS, without LPS and sample with LPS.

**B)**

Cell was collected into 2xsample reducing buffer and incubated at boiling water for 5 minutes. 20μl of sample was loaded onto 8% SDS-PAGE, followed by electric transfer to nitrocellulose membrane (Bio-Rad Laboratory). Membrane was blocked 1 h at room temperature with 3% skimmed milk powder in 50mM Tris buffer (pH7.5) containing 150mM NaCl and 0.05% Tween-20. Membrane was then incubated at 4°C overnight with mouse anti-iNOS antibody (Pharmingen Transduction Laboratories) and mouse anti-α-tubulin antibody (Sigma) in 50mM Tris buffer with 150mM NaCl (pH7.5). Membrane was then incubated with goat anti-mouse IgG conjugated with horse radish peroxidase (Pharmingen Transduction Laboratories) for 1 h at room temperature. 4-chloro-1-naphthol and hydrogen peroxide were used for the visualization of target protein [19] (Bollag et al, 1996).

In addition to reactive oxygen species, reactive oxygen species such as nitric oxide and its metabolite peroxynitrite are considered mutagenic [21](Keeper and Wink, 1996).There are two types of nitric oxide synthesis (NOS) in the mammalian cell, i.e., constituted NOS (cNOS) and inducible NOS (iNOS), the latter being most likely to be activated by endotoxins such as lipopolysaccharide (LPS) and cytokines. The iNOS is not present in most cells under normal conditions, however, following the appropriate stimulus such as LPS and cytokines, it is rapidly induced and responsible for the large quantity of nitric oxide. The incubation of bacterial lipopolysaccharide with macrophage provided a quick way to evaluate the generation of nitric oxide from the inducible nitric oxide synthase. Leeuwenburgh *et al* [22](1997) has reported that LDL from aortic atherosclerotic intima contained significantly higher 3-nitrotryosine than plasma LDL, indicating the implication of reactive nitric species in aortic LDL oxidation and atherosclerosis. Further, it has been shown that iNOS inhibitor N-iminoethyl-L-lysine limited the progression of preexisting atherosclerosis in hypercholesterolemic rabbits thus suggesting that inhibition of iNOS might be beneficial for the preventing the progression of atherosclerosis.

Large amount of nitric oxide expression was made possible by using mouse macrophage cell line RAW264.7 co-incubated with bacterial LPS. The measurement of nitric oxide was indirectly measured by its metabolite nitrite in the supernatant of cell cultural media by Griess reagent. Nitrite was found in the cultural media at 25μM which was higher compared to the macrophage cell without LPS treatment, indicating that nitric oxide formation was boosted when RAW264.7 cell was stimulated by bacterial lipopolysaccharide.

The addition of black rice extract significantly reduced the production of nitrite, indicating the suppression of nitric oxide in the activated macrophage cells (Figure 18). It is also notable that the inhibition of nitric oxide production was not due to any cytotoxicity, since the cell viability was normal with the addition of black rice extract (Figure 18).The inhibition of nitric oxide was also found for cyanidin-3-glucoside and peonidin-3-glucoside. Under the same working condition, inhibition of nitric oxide in LPS-activated RAW264.7 cell by cyanidin-3-glucoside and peonidin-3-glucoside is shown in Table 5.

**Table 5**. Inhibition percentage of nitric oxide generated in the LPS-activated mouse macrophage cell RAW264.7 (mean±SD)

| | Cyanidin-3-glucoside | Peonidin-3-glucoside |
|---|---|---|
| 1μM | 12.7±2.7 | 14.4±0.6 |
| 10μM | 23.1±0.9 | 22.3±3.7 |
| 100μM | 72.9±4.8 | 35.0±2.5 |

It was noteworthy that cyanidin-3-glucoside revealed a higher inhibition of nitric oxide formation than that of peonidin-3-glucoside at 100μM.

In our present study, it is clear that the addition of LPS to the RAW264.7 cell significantly induces the expression of iNOS protein as demonstrated by the western blotting (Figure 19). The addition of black rice extract in this cell culture model indicated that the expression of iNOS protein (MW 130kDa) was suppressed at a concentration dependent manner (Figure 19). In addition, cell lysate blotted with anti-α-tubulin antibody showed that this housekeeping protein remained unchanged with or without LPS and samples treatments, thus suggesting that black rice, specifically suppresses the inducible nitric oxide synthase express in the macrophage, thus reduces the nitric oxide production under this condition.

**Example 12 Reduction in the Development of Atherosclerosis in Apo-E Mice**

ApoE-deficient mice on a C57BL/6J background were purchased from Jackson Laboratories (Bar Harbor,Maine U.S.A), and were bred and maintained under conventional housing conditions. C57BL/6J mice were from the Animal Center of Sun-Yat Sen University of Medical Sciences.

A total 45 male apoE - deficient mice of 4wk age were randomly divided into 3 groups of 15 each group and matched for body weight. The mice received a purified diet based on the AIN-93G formulation (summarized in Table 6) and each group consumed one of the following diet:AIN-93G purified diet(Positive Group); AIN-93G purified diet with 5g/100g of the extract of the present invention: black rice fraction(BRF Group); AIN-93G purified diet with 5g/100g white rice fraction(WRF Group). 15 male C57BL/6J mice aged 4wk old as control(Control Group) were fed AIN-93G purified diet.

The contents of protein,fat and energy in the different diets were adjusted to the same level by adding casein

and soybean oil (Table 7). The components of the BRF and WRF were listed in Table 5. All the groups were housed in plastic cages with stainless steel grid tops.Food and distilled water given ad libitum. The experiment lasted 16 weeks.The average amount of dietary intake in each mouse was ≈2.8g/d in four groups. The mice were weighed twice every week during the experiment.

At the end of the experiment, all mice were deprived of food overnight and kindly killed by withdrawing blood from retro-orbital plexus under anaesthesia. Serum was prepared by low speed centrifugation at 2800×g for 20 min at 4°C and used to determine the levels of serum lipids.

Table 6 AIN-93 Purified Diets for Laboratory Rodents

| Ingredient | g/kg diet |
|---|---|
| Cornstarch | 397.486 |
| Casein | 200 |
| Dextrinized Cornstarch | 132 |
| Sucrose | 100 |
| Soybean Oil(no additive) | 70 |
| Fiber | 50 |
| Mineral mix(AIN-93G-MX)[1] | 35 |
| Vitamin mix(AIN-93-MX)[2] | 10 |
| L-Cysteine | 3 |
| Choline bitartrate (41.1 % choline)[3] | 2.5 |
| Tert-butylhydroquinone | 0.014 |

[1,2] ICN Company,U.S.A
[3]Based on the molecular weight of the free base.

Table 7 Major nutritients of of different dietary groups*

| Ingredient | Control Group | Positive Group | BRF Group | WRF Group |
|---|---|---|---|---|
| Energy (kcal/kg diet) | 3766.0 | 3766.0 | 3766.0 | 3766.0 |
| Carbohydrate (g/kg diet) | 629.486 | 629.486 | 629.486 | 629.486 |
| Protein (g/kg diet) | 200 | 200 | 200 | 200 |
| Total fat (g/kg diet) | 70 | 70 | 70 | 70 |

*Control group:C57BL/6J mice fed the AIN-93G purified diet Positive Group:apoE-deficient mice fed the AIN-93G purified diet
BRF Group:apoE-deficient mice fed the AIN-93G purified diet with 5g/100g black rice fraction
WRF Group: apoE-deficient mice fed the AIN-93G purified diet with 5g/100g white rice fraction

Table 8 Components of the black and white rice fraction

| Ingredient | Black rice fraction | White rice fraction |
|---|---|---|
| | Units/100g | Units/100g |
| Protein, g | 13.90 | 12.20 |
| Fat, g | 13.20 | 14.10 |
| Carbohydrate, g | 47.36 | 50.95 |
| Moisture, g | 9.80 | 7.96 |
| Fiber, g | 8.32 | 7.04 |
| Salt mixture, mg | 7420 | 7750 |
| P | 1694.10 | 1542.50 |
| Ca | 60.20 | 45.30 |

(continued)

| Ingredient | Black rice fraction | White rice fraction |
|---|---|---|
| | Units/100g | Units/100g |
| K | 673.70 | 624.60 |
| Mg | 79.40 | 80.40 |
| Na | 2.11 | 4.35 |
| Fe | 16.46 | 6.30 |
| Zn | 8.96 | 4.92 |
| Mn | 11.67 | 7.93 |
| Mo | 0.33 | 0.28 |
| Cu | 1.49 | 0.91 |
| Se | 0.15 | 0.06 |
| Vitamin, mg | | |
| Vitamin B1 | 2.30 | 1.20 |
| Vitamin B2 | 0.40 | 0.14 |
| Vitamin E | 0.60 | 0.03 |
| Nicotinic acid | 21.00 | 13.00 |
| Total flavonoids, mg | 6.4 | 1.2 |

Serum lipid profile

Serum total cholesterol (TC), low density lipoprotein cholesterol (LDL-C), high density lipoprotein cholesterol (HDL-C) were measured by using a Hitachi Automatic Analyzer (Tokyo,Japan). Serum TC was determined by using a cholesterol esterase and cholesterol oxidase assay. Serum concemtrations of HDL-C were assayed by the same method. Serum LDL concentrations were calculated according to Friedwald formula.

Assessment of Atherosclerosis

Quantification of atherosclerosis fatty streaks was done by calculating the lesion size in the aortic sinus as previously described (18). Briefly,the heart and upper section of the aorta were removed from the animals and the peripheral fat cleaned carefully. The upper section was embedded in O.C.T compound and frozen at -20°C. Every other section (10um thick) throughout the aortic sinus (400um) was taken for analysis. The distal portion of the aortic sinus was recognized by the three valve cusps which are the junctions of the aorta and the heart. Cryostat sections were stained with oil red O and counter-stained with hematoxylin. Each section of the aortic valve was evaluated for oil-red O staining area by capturing images directly from an RGB camera attached to an Olympus BX-50 light microscope and displaying them on a Trinitron™ RGB monitor. Image analysis was determined using Optimas™ 4.1 software (Image Processing Solutions). Results were expressed as the percent of the total cross-sectional vessel wall area (normal+diseased area/section, excluding the lumen) stained with oil red O.

Statistical analysis

Results are expressed as means $\pm$ SD, and the differences were determined by one-way ANOVA coupled with the Student-Newman-keuls (SNK) multiple comparison test. Differences with $P < 0.05$ were considered significant.

Results

Body Weights

Fifteen mice in each group were initiated onto the experimental protocol. Initial mice body weights for the experiment were 17$\pm$1 g (mean$\pm$SD). Final average body weights were 25 to 27g. No significant difference in body weights was observed during the experimental period (Table 9) (Figure 16).

Table 9 Body weights of mice in different groups fed AIN-93G diet or AIN-93G diet containing WRF,BRF during 16wk

| Group | N | 4wk | 8wk | 12wk | 16wk |
|---|---|---|---|---|---|
| Control Group | 15 | 17.07±1.08 | 22.41±2.13 | 25.67±2.34 | 26.45±2.63 |
| Positive Group | 15 | 17.16±1.04 | 21.13±3.14 | 23.77±2.75 | 24.47±2.85 |
| BRF Group | 15 | 17.11±1.05 | 21.35±3.31 | 24.73±3.24 | 25.90±3.13 |
| WRF Group | 15 | 17.18±1.07 | 21.83±2.72 | 24.38±3.07 | 26.18±3.00 |

Serum Cholesterol Levels

Serum TC,LDL-C,HDL-C and LDL/HDL in control group differed from the other three groups(P<0.05).The levels of TC,LDL-C and LDL/HDL in BRF group were lower compared with positive group and WRF group(P<0.05),and there were no differences between positive group and WRF group. Although both BRF and WRF groups had higher levels HDL-C compared with positive group, the BRF group had lower LDL/HDL (Table 10).

Table 10 Serum lipid concentrations in mice fed AIN-93G diet or AIN-93G diet containing WRF,BRF for 16wk*

| Group | n | TC(nimol/l) | LDL-C(mmol/l) | HDL-C(mmol/l) | LDUHDL |
|---|---|---|---|---|---|
| Control Group | 10 | 2.881±0.758a | 0.280±0.083a | 1.776±0.439a | 0.160±0.038 a |
| Positive Group | 10 | 15.682±2.644 b | 1.427±0.420b | 2.546±0.442b | 0.570±0.188 b |
| BRF Group | 10 | 12.118±1.833 c | 0.747±0.251c | 2.659±0.398c | 0.343±0.170 c |
| WRF Group | 10 | 17.240±3.646 b | 1.315±0.562b | 2.645±0.663c | 0.450±0.179 b |

* Values are means ± S.D. Values without common letter in colomn are significiantly different, P<0.05. Abbreviations used: TC, total cholesterol; LDL, low density lipoprotein cholesterol; HDL, high density lipoprotein cholesterol; C, cholesterol;

Development of Atherosclerosis in the Aortic Sinus

After 16 weeks intervention,there were no visible atherosclerotic plaques in the aortic sinus of the control group fed the AIN-93G diet. But atherosclerotic plaques of various degrees were visible in aortic sinus in other three groups. Plaque was much more severe in positive group and WRF group than in BRF group (Table 11)(Figure 17).

The average plaque area in mice of BRF group was lower than that of positive group and WRF group (P<0.01). The plaque area of aortic sinus in mice fed BRF was 48.42% and 46.08% less compared to positive group and WRF group respectively. There was no significant difference in plaque areas of mice in positive group and WRF group.

Table 11 Atherosclerotic plaque of aortic sinus in mice fed AIN-93G diet or AIN-93G diet containing WRF,BRF after 16wk*

| Group | n | Atherosclerotic Area(%) |
|---|---|---|
| Control Group | 15 | 0a |
| Positive Group | 15 | 26.25±9.2b |
| BRF Group | 15 | 13.54±4.1c |
| WRF Group | 15 | 25.11±7.15b |

*Values without common letters in column are significiantly different, p<0.01.

**REFERENCES**

1. Breslow 1997 Nat.Med 3:600-601

2.Law et al Br. Med. J. 1994; 308:363-366

3. Law et al. Br. Med. J. 1994; 308:367-373

*4.* Circulation 1990; 81:1721-1733

5. Havel R.J., Rapaport E.. New England Journal of Medicine, 1995; 332:1491-1498

6. Barker and Rye Atherosclerosis 1996; 121:1-12

7. Braunwald E. 1997 N. Engl. J. Med. 340:115-126

8. Ross R. 1999 N. Engl. J. Med. 340:115-126

9. Liao D.F. et al.2000 J. Biol. Chem. 275:189-196

10. Shackelford R.E. et al. 2000 Free Rad. Biol. Med. 28: 1387-1404

11. Giugliano D. 2000 Nutr. Metab. Cardiovasc. Dis. 10:38-44

12a. Kunsch C. and Medford R.M. 1999 Circ. Res. 85:753-766

12b Yuan, Y.V., Kitts, D.D. and Godin, D.V. 1997. Influence of dietary cholesterol and fat source on atherosclerosis in Japanese quail. Brit. J. Nutr. 78: 993-1014

13. Genest J. 1986 Can. J. Physiol. Pharmacol. 64:836-839

14.Simmons D. and Willaims R. 1997 Br. J. Nutr. 78:5-14

15. Ling et al. Biomedical and Molecular Action of Nutrients 2001

16. Liposome Drug Delivery Systems, Technomic Publishing Co. Inc., Lancaster, PA 1993

17. Pharmaceutical Technology: Liposomes as Drug Delivery Systems Parts I , II and III, October 1992, November 1992 and January 1993 respectively

18. Paigen B et al, 1987 Atherosclerosis 68:231

19. Schneider, J.E.; Browning, M.M.; Floyd, R.A. Ascorbate/iron mediation of hydroxyl free radical damage to PBR322 plasmid DNA. Free Rad Biol Med 1988, 5: 287-295.

20. Kitts, D.D.; Yuan, Y.V.; Wijewickreme, A.N.; Thompson, L.U. Antioxidant activity of the flaxseed lignan secoisolariciresinol diglycoside and its mammalian lignan metabolites enterodiol and enterolactone. Mol Cell Biochem.1999, 202: 91-101.

21. Keefer, L.K.; Wink, K. DNA damage and nitric oxide. Adv Exp Med Biol. 2002, 387: 177-185.

22. Leeuwenburgh, C.; Hardy, M.M.; Hazen, S.L.; Wagner, P.; Oh-ishi, S.; Steinbrecher, U.P.; Heinecke, J. Reactive nitrogen intermediates promote low density lipoprotein oxidation in human atherosclerotic initima. J Biol Chem 1997, 272: 1433-1438.

## Claims

1. The use of a composition comprising cyanidin-3-O-glucoside and peonidin-3-O-glucoside in the manufacture of a medicament or nutraceutical for reducing LDL cholesterol.

2. The use of claim 1 in which the ratio of cyanidin-3-O-glucoside to peonidin -3-O-glucoside is in the range 10:1 to 1:1.

3. The use of claim 1 in which the ratio of cyanidin-3-O-glucoside to peonidin-3-O-glucoside is in the range 3.5:1 to 6,5:1

4. The use of claim 1 in which the ratio of cyanidin-3-O-glucoside to peonidin -3-O-glucoside is 4:1.

5. The use of claim 1 wherein the medicament or nutraceutical additionally comprises one or more anti-oxidants.

6. The use of claim 5 wherein the anti-oxidants are selected from the group consisting of vitamin E, beta-carotein, enzymatic superoxide dismutase, catalase, glutathione peroxidase, glutathione reductase, tea catechins, chelating agents such as citric acid, EDTA, phenylalanine, phosphoric acid, tartaric acid and tryptophane, preferentially oxidized compounds such as ascorbic acid, sodium bisulfite and sodium sulfite, water soluble chain terminators such as thiols and lipid soluble chain terminators such as alkyl gallates, ascorbyl palmitate, t-butyl hydroquinone, butylated hydroxyanisole, butylated hydroxytoluene, hydroquinone, nordihydroguaiaretic acid and alpha-tocopherol.

7. The use of claim 1 wherein the medicament or nutraceutical additionally comprises one or more phytosterols.

8. The use of claim 1 wherein the medicament or nutraceutical additionally comprises one or more phytostanols.

9. The use of claim 1 wherein the treatment prevents, reduces, eliminates or ameliorates a condition disease or disorder which has as its basis or which is exacerbated by an excess of LDL.

10. The use of a composition comprising an extract of the outer layer of dehulled black rice, said extract comprising cyanidin-3-O-glucoside and peonidin-3-O-glucoside in the manufacture of a medicament or nutraceutical for reducing LDL cholesterol.

11. The use of claim 10 wherein the extract is prepared by the following steps:

    a) separating an outer layer, predominantly comprising the pericarp, tegmen, and aleurone layers, from a starchy endosperm in de-hulled black rice;
    b) adding a solution of at least one organic solvent and an acid to the separated outer layer sufficient to achieve a pH of between 1 and 4;
    c) filtering and removing the solvent and the acid from the separated outer layer to produce a pigment fraction;
    d) separating constituents of the pigment fraction; and
    e) collecting an anthocyanin composition, comprising cyanidin-3-O-glucoside and peonidin-3-O-glucoside, therefrom.

12. The use of claim 11 wherein the outer layer is physically separated from the starchy endosperm at step a).

13. The use of claim 11 wherein the outer layer is physically separated from the starchy endosperm at step a) by milling the de-hulled black rice.

14. The use of claim 11 wherein the organic solvent is selected from the group consisting of alcohols, ketones, hydrocarbons and water.

15. The use of claim 11 wherein the organic solvent is a ketone having the structure $RCOR^1$ where R and $R^1$ are $C_1$-$C_6$ alkyl groups.

16. The use of claim 11 wherein the organic solvent is a $C_5$-$C_{10}$ hydrocarbon.

17. The use of claim 11 wherein the organic solvent is an alcohol selected from the group consisting of the general structures R-CHOHR, R-$CH_2$OH, and RCOH where R is a $C_1$-$C_4$ alkyl group.

18. The use of claim 11 wherein the acid is selected from the group consisting of hydrochloric acid, acetic acid, citric acid, low concentration sulphuric acid and tartaric acid.

19. The use of claim 11 wherein the separated outer layer is soaked in the solvent for a period of between 1 and 10 hours.

20. The use of claim 11 wherein the solvent and acid are removed at step c) by evaporation.

21. The use of claim 11 wherein the constituents of the pigment fraction are separated at step d) by fractionation.

22. The use of claim 10 in which the ratio of cyanidin-3-O-glucoside to peonidin-3-O-glucoside in the extract is in the range 10:1 to 1:1.

23. The use of claim 10 in which the ratio of cyanidin-3-O-glucoside to peonidin-3-O-glucoside in the extract is in the range 3.5:1 to 6,5:1

24. The use of claim 10 in which the ratio of cyanidin-3-O-glucoside to peonidin-3-O-glucoside in the extract is 4:1.

25. The use of claim 10 wherein the medicament or nutraceutical additionally comprises one or more anti-oxidants.

26. The use of claim 10 wherein the medicament or nutraceutical additionally comprises one or more phytosterols.

**Patentansprüche**

1. Verwendung einer Zusammensetzung, umfassend Cyanidin-3-O-glucosid und Peonidin-3-O-glucosid in der Herstellung eines Arzneimittels oder Nutrazeutikums zur Senkung des LDL-Cholesterins.

2. Verwendung nach Anspruch 1, wobei das Verhältnis von Cyanidin-3-O-glucosid zu Peonidin-3-O-glucosid im Bereich von 10:1 bis 1:1 liegt.

3. Verwendung nach Anspruch 1, wobei das Verhältnis von Cyanidin-3-O-glucosid zu Peonidin-3-O-glucosid im Bereich von 3,5:1 bis 6,5:1 liegt.

4. Verwendung nach Anspruch 1, wobei das Verhältnis von Cyanidin-3-O-glucosid zu Peonidin-3-O-glucosid 4:1 beträgt.

5. Verwendung nach Anspruch 1, wobei das Arzneimittel oder Nutrazeutikum zusätzlich ein oder mehr Antioxidanz (ien) umfasst.

6. Verwendung nach Anspruch 5, wobei die Antioxidanzien aus der Gruppe ausgewählt sind, bestehend aus: Vitamin E, β-Carotin, enzymatischer Superoxiddismutase, Katalase, Glutathionperoxidase, Glutathionreduktase, Teekatechinen, Chelatbildnern, wie zum Beispiel Citronensäure, EDTA, Phenylalanin, Phosphorsäure, Weinsäure und Tryptophan, bevorzugt oxidierten Verbindungen, wie zum Beispiel Ascorbinsäure, Natriumbisulfit und Natriumsulfit, wasserlöslichen Kettenabbruchmitteln, wie zum Beispiel Thiolen und lipidlöslichen Kettenabbruchmitteln, wie zum Beispiel Alkylgallaten, Ascorbylpalmitat, t-Butylhydrochinon, butyliertem Hydroxyanisol, butyliertem Hydroxytoluen, Hydrochinon, Nor-dihydroguajaretsäure und α-Tocopherol.

7. Verwendung nach Anspruch 1, wobei das Arzneimittel oder Nutrazeutikum zusätzlich ein oder mehr Phytosterol(e) umfasst.

8. Verwendung nach Anspruch 1, wobei das Arzneimittel oder Nutrazeutikum zusätzlich ein oder mehr Phytostanole umfasst.

9. Verwendung nach Anspruch 1, wobei die Behandlung einen Zustand, eine Erkrankung oder Störung verhindert, reduziert, eliminiert oder lindert, dem/der ein LDL-Überschuss zugrunde liegt oder der/die **dadurch** exazerbiert wird.

10. Verwendung einer Zusammensetzung, umfassend einen Extrakt von der Außenschicht von geschältem Schwarzreis, wobei der Extrakt Cyanidin-3-O-glucosid und Peonidin-3-O-glucosid in der Herstellung eines Arzneimittels oder

Nutrazeutikums zur Senkung des LDL-Cholesterins umfasst.

**11.** Verwendung nach Anspruch 10, wobei der Extrakt durch die folgenden Schritte hergestellt wird:

a) Abtrennen einer überwiegend die Pericarp-, Tegmen- und Aleuronschichten umfassenden Außenschicht von einem stärkehaltigen Endosperm im geschälten Schwarzreis;
b) Zufügen einer Lösung von mindestens einem organischen Lösungsmittel und einer Säure zur abgetrennten Außenschicht, die zum Erreichen eines pH zwischen 1 und 4 ausreichend ist;
c) Filtrieren und Entfernen des Lösungsmittels und der Säure aus der abgetrennten Außenschicht zur Herstellung einer Pigmentfraktion;
d) Abtrennen von Bestandteilen der Pigmentfraktion; und
e) Sammeln einer Anthocyaninzusammensetzung, umfassend C yanidin-3-O-glucosid und Peonidin-3-O-glucosid, daraus.

**12.** Verwendung nach Anspruch 11, wobei die Außenschicht vom stärkehaltigen Endosperm in Schritt a) physikalisch getrennt wird.

**13.** Verwendung nach Anspruch 11, wobei die Außenschicht vom stärkehaltigen Endosperm in Schritt a) durch Vermahlen des geschälten Schwarzreises physikalisch getrennt wird.

**14.** Verwendung nach Anspruch 11, wobei das organische Lösungsmittel aus der Gruppe ausgewählt ist, die aus Alkoholen, Ketonen, Kohlenwasserstoffen und Wasser besteht.

**15.** Verwendung nach Anspruch 11, wobei das organische Lösungsmittel ein Keton mit der Struktur $RCOR^1$ ist, worin R und $R^1$ $C_1$-$C_6$-Alkylgruppen sind.

**16.** Verwendung nach Anspruch 11, wobei das organische Lösungsmittel ein $C_5$-$C_{10}$-Kohlenwasserstoff ist.

**17.** Verwendung nach Anspruch 11, wobei das organische Lösungsmittel ein Alkohol ist, der aus der Gruppe ausgewählt ist, die aus den allgemeinen Strukturen R-CHOHR, R-$CH_2$OH und RCOH besteht, worin R eine $C_1$-$C_4$-Alkylgruppe ist.

**18.** Verwendung nach Anspruch 11, wobei die Säure aus der Gruppe ausgewählt ist, die aus Salzsäure, Essigsäure, Citronensäure, niedrig konzentrierter Schwefelsäure und Weinsäure besteht.

**19.** Verwendung nach Anspruch 11, wobei die abgetrennte Außenschicht für eine Zeitdauer zwischen 1 und 10 Stunden im Lösungsmittel eingeweicht wird.

**20.** Verwendung nach Anspruch 11, wobei das Lösungsmittel und die Säure im Schritt c) durch Verdampfung entfernt werden.

**21.** Verwendung nach Anspruch 11, wobei die Bestandteile der Pigmentfraktion im Schritt d) durch Fraktionierung abgetrennt werden.

**22.** Verwendung nach Anspruch 10, worin das Verhältnis von Cyanidin-3-O-glucosid zu Peonidin-3-O-glucosid im Extrakt im Bereich von 10:1 bis 1:1 liegt.

**23.** Verwendung nach Anspruch 10, worin das Verhältnis von Cyanidin-3-O-glucosid zu Peonidin-3-O-glucosid im Extrakt im Bereich von 3,5:1 bis 6,5:1 liegt.

**24.** Verwendung nach Anspruch 10, worin das Verhältnis von Cyanidin-3-O-glucosid zu Peonidin-3-O-glucosid im Extrakt 4:1 beträgt.

**25.** Verwendung nach Anspruch 10, wobei das Arzneimittel oder Nutrazeutikum zusätzlich ein oder mehr Antioxidanz (ien) umfasst.

**26.** Verwendung nach Anspruch 10, wobei das Arzneimittel oder Nutrazeutikum zusätzlich ein oder mehr Phytosterol (e) umfasst.

## Revendications

1. Utilisation d'une composition comprenant du cyanidine-3-O-glucoside et du péonidine-3-O-glucoside dans la fabrication d'un médicament ou d'un produit nutri-pharmaceutique pour réduire le cholestérol LDL.

2. Utilisation selon la revendication 1, dans laquelle le rapport cyanidine-3-O-glucoside/péonidine-3-O-glucoside est compris entre 10/1 et 1/1.

3. Utilisation selon la revendication 1, dans laquelle le rapport cyanidine-3-O-glucoside/péonidine-3-O-glucoside se situe entre 3,5/1 et 6,5/1.

4. Utilisation selon la revendication 1, dans laquelle le rapport cyanidine-3-O-glucoside/péonidine-3-O-glucoside est égal à 4/1.

5. Utilisation selon la revendication 1, dans laquelle le médicament ou le produit nutri-pharmaceutique comprend, en outre, un ou plusieurs anti-oxydants.

6. Utilisation selon la revendication 5, dans laquelle les anti-oxydants sont sélectionnés dans le groupe constitué par la vitamine E, le bêta-carotène, la superoxyde-dismutase enzymatique, la catalase, la glutathione-peroxydase, la glutathione-réductase, les catéchines du thé, des agents chélateurs tels que l'acide citrique, l'EDTA, la phénylalanine, l'acide phosphorique, l'acide tartrique et le tryptophane, de préférence des composés oxydés tels que l'acide ascorbique, le bisulfite de sodium et le sulfite de sodium, des terminateurs de chaîne hydrosolubles tels que les thiols et des terminateurs de chaîne liposolubles tels que les alkyl-gallates, le palmitate d'ascorbyle, l'hydroquinone t-butylique, l'hydroxyanisole butylée, l'hydroxytoluène butylé, l'hydroquinone, l'acide nordihydroguaiarétique et l'alpha-tocophérol.

7. Utilisation selon la revendication 1, dans laquelle le médicament ou le produit nutri-pharmaceutique comprend, en outre, un ou plusieurs phytostérols.

8. Utilisation selon la revendication 1, dans laquelle le médicament ou le produit nutri-pharmaceutique comprend, en outre un ou plusieurs phytostanols.

9. Utilisation selon la revendication 1, dans laquelle le traitement empêche, réduit, élimine ou amoindrit un état pathologique ou un trouble qui découle de ou qui est exacerbé par un excès de LDL.

10. Utilisation d'une composition comprenant un extrait de la couche externe du riz noir décortiqué, ledit extrait comprenant du cyanidine-3-O-glucoside et du péonidine-3-O-glucoside dans la fabrication d'un médicament ou d'un produit nutri-pharmaceutique pour réduire le cholestérol LDL.

11. Utilisation selon la revendication 10, dans laquelle l'extrait est préparé selon les étapes suivantes :

    a) la séparation entre une couche externe, principalement composée des couches de péricarpe, de tegmen et d'aleurone, et un albumen amylacé dans du riz noir décortiqué ;
    b) l'addition d'une solution d'au moins un solvant organique et d'un acide à la couche externe séparée, en quantités suffisantes pour obtenir un pH compris entre 1 et 4 ;
    c) la filtration et l'extraction du solvant et de l'acide de la couche externe séparée pour produire une fraction pigmentaire ;
    d) la séparation des constituants de la fraction pigmentaire ; et
    e) le recueil d'une composition anthocyanique, comprenant le cyanidine-3-O-glucoside et le péonidine-3-O-glucoside de celle-ci.

12. Utilisation selon la revendication 11, dans laquelle la couche externe est physiquement séparée de l'albumen amylacé à l'étape a).

13. Utilisation selon la revendication 11, dans laquelle la couche externe est physiquement séparée de l'albumen amylacé à l'étape a) par mouture du riz noir décortiqué.

14. Utilisation selon la revendication 11, dans laquelle le solvant organique est sélectionné dans le groupe constitué

par les alcools, les cétones, les hydrocarbures et l'eau.

**15.** Utilisation selon la revendication 11, dans laquelle le solvant organique est une cétone ayant pour structure $RCOR^1$, dans laquelle R et $R^1$ sont des groupes alkyles $C_1$-$C_6$.

**16.** Utilisation selon la revendication 11, dans laquelle le solvant organique est un hydrocarbure $C_5$-$C_{10}$.

**17.** Utilisation selon la revendication 11, dans laquelle le solvant organique est un alcool sélectionné dans le groupe constitué par les structures générales R-CHOHR, R-$CH_2$OH et RCOH, dans lesquelles R est un groupe alkyle $C_1$-$C_4$.

**18.** Utilisation selon la revendication 11, dans laquelle l'acide est sélectionné dans le groupe constitué par l'acide chlorhydrique, l'acide acétique, l'acide citrique, l'acide sulfurique et l'acide tartrique à faible concentration.

**19.** Utilisation selon la revendication 11, dans laquelle la couche externe séparée est imprégnée de solvant pendant une période comprise entre 1 et 10 heures.

**20.** Utilisation selon la revendication 11, dans laquelle le solvant et l'acide sont éliminés par évaporation à l'étape c).

**21.** Utilisation selon la revendication 11, dans laquelle les constituants de la fraction pigmentaire sont séparés par fractionnement à l'étape d).

**22.** Utilisation selon la revendication 10, dans laquelle le rapport cyanidine-3-O-glucoside/péonidine-3-O-glucoside dans l'extrait est compris entre 10/1 et 1/1.

**23.** Utilisation selon la revendication 10, dans laquelle le rapport cyanidine-3-O-glucoside/péonidine-3-O-glucoside dans l'extrait est compris entre 3,5/1 et 6,5/1.

**24.** Utilisation selon la revendication 10, dans laquelle le rapport cyanidine-3-O-glucoside/péonidine-3-O-glucoside dans l'extrait est égal à 4/1.

**25.** Utilisation selon la revendication 10, dans laquelle le médicament ou le produit nutri-pharmaceutique comprend, en outre, un ou plusieurs anti-oxydants.

**26.** Utilisation selon la revendication 10, dans laquelle le médicament ou le produit nutri-pharmaceutique comprend, en outre, un ou plusieurs phytostérols.

Figure 1

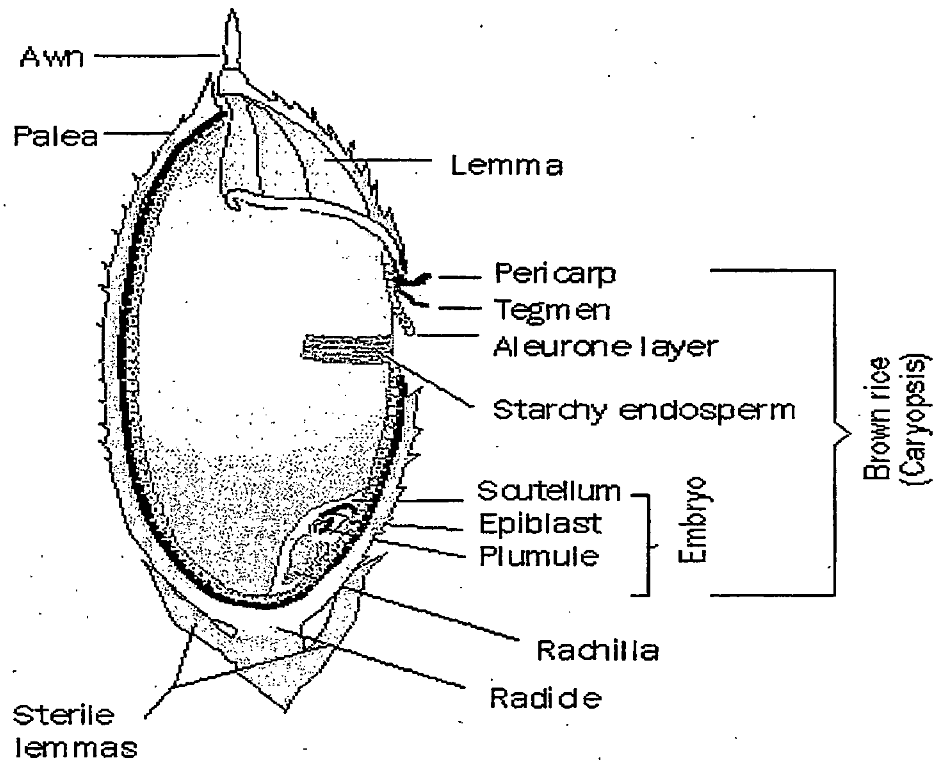
Awn
Palea
Lemma
Pericarp
Tegmen
Aleurone layer
Starchy endosperm
Scutellum
Epiblast
Plumule
Embryo
Brown rice (Caryopsis)
Rachilla
Radicle
Sterile lemmas

Figure 2

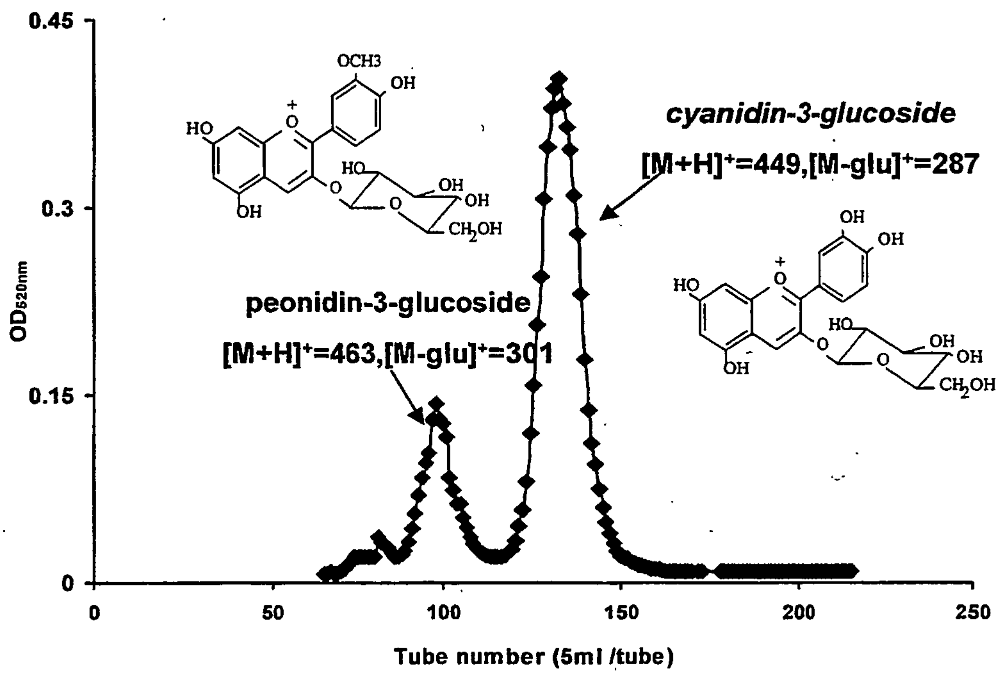
0.45
0.3
0.15
0
OD520nm
cyanidin-3-glucoside
[M+H]+=449,[M-glu]+=287
peonidin-3-glucoside
[M+H]+=463,[M-glu]+=301
0
50
100
150
200
250
Tube number (5ml /tube)

Figure 3

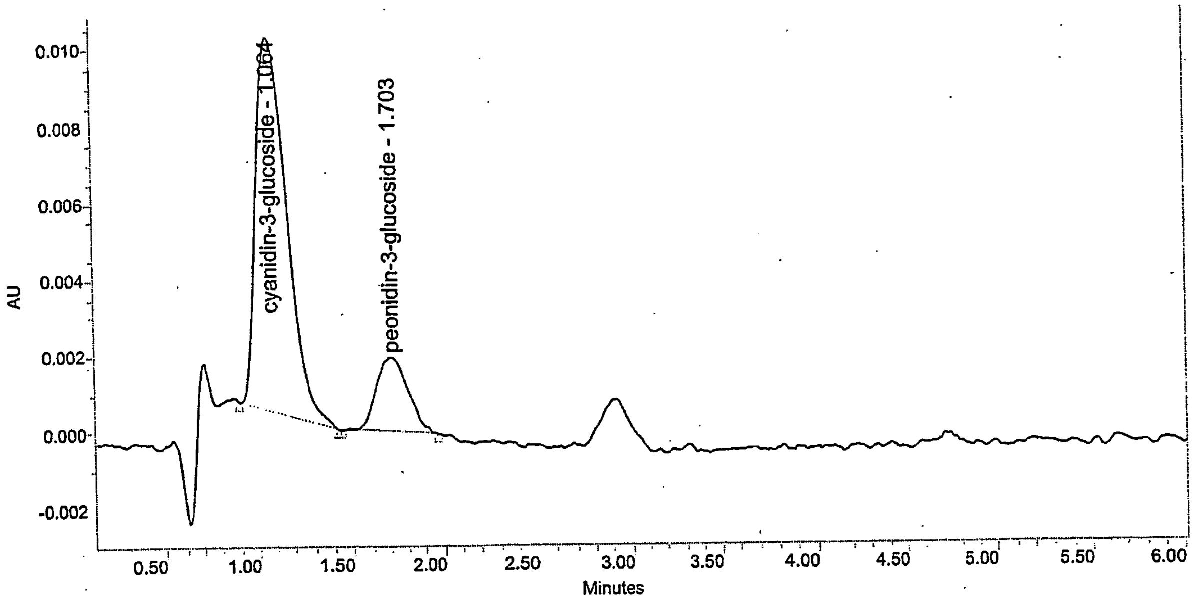


| | Peak Name | RT | Area | % Area | Height |
|---|---|---|---|---|---|
| 1 | cyanidin-3-glucoside | 1.064 | 113872 | 82.15 | 9757 |
| 2 | peonidin-3-glucoside | 1.703 | 24742 | 17.85 | 1973 |

Figure 4

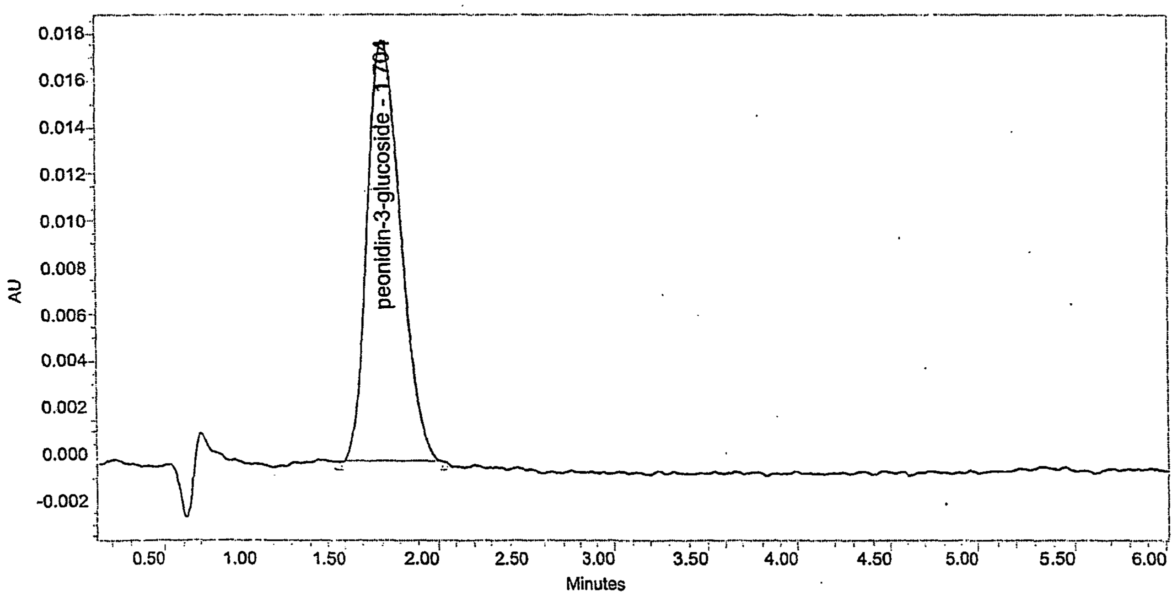


| | Peak Name | RT | Area | % Area | Height |
|---|---|---|---|---|---|
| 1 | cyanidin-3-glucoside | 1.083 | | | |
| 2 | peonidin-3-glucoside | 1.704 | 227249 | 100.00 | 18020 |

Figure 5

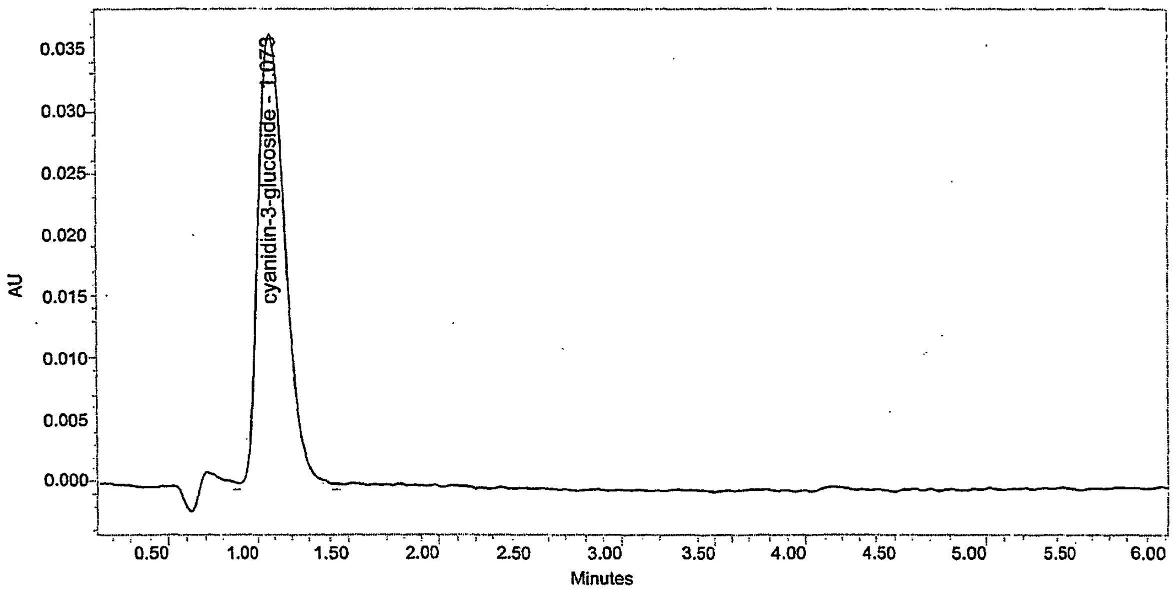


| | Peak Name | RT | Area | % Area | Height |
|---|---|---|---|---|---|
| 1 | cyanidin-3-glucoside | 1.073 | 386101 | 100.00 | 36332 |
| 2 | peonidin-3-glucoside | 1.744 | | | |

Figure 6

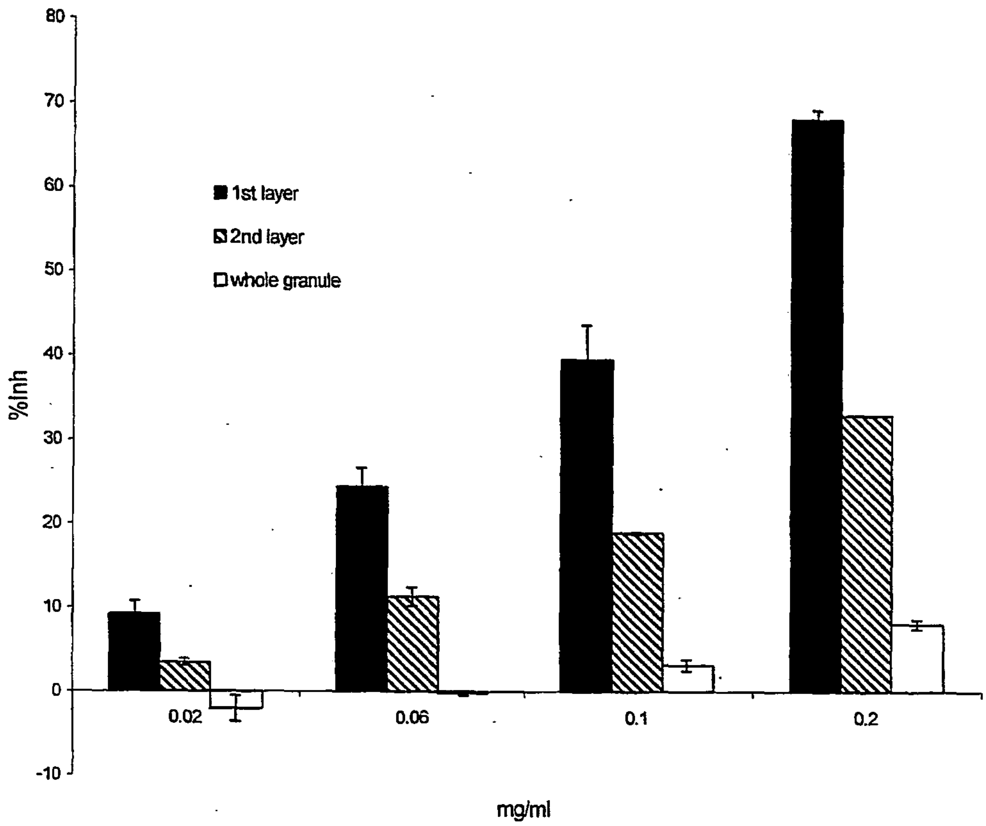
80
70
60
50
40
30
20
10
0
-10
%Inh
1st layer
2nd layer
whole granule
0.02
0.06
0.1
0.2
mg/ml

Figure 7

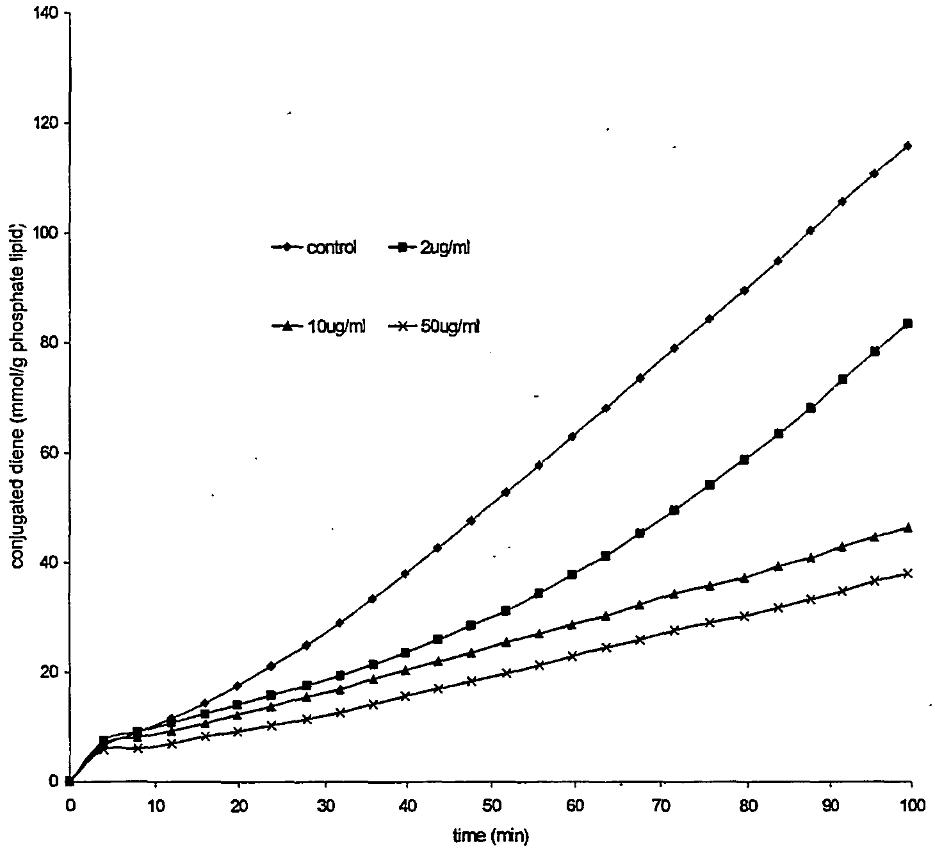
140
120
100
80
60
40
20
0
conjugated diene (mmol/g phosphate lipid)
control
2ug/ml
10ug/ml
50ug/ml
0
10
20
30
40
50
60
70
80
90
100
time (min)

Figure 8

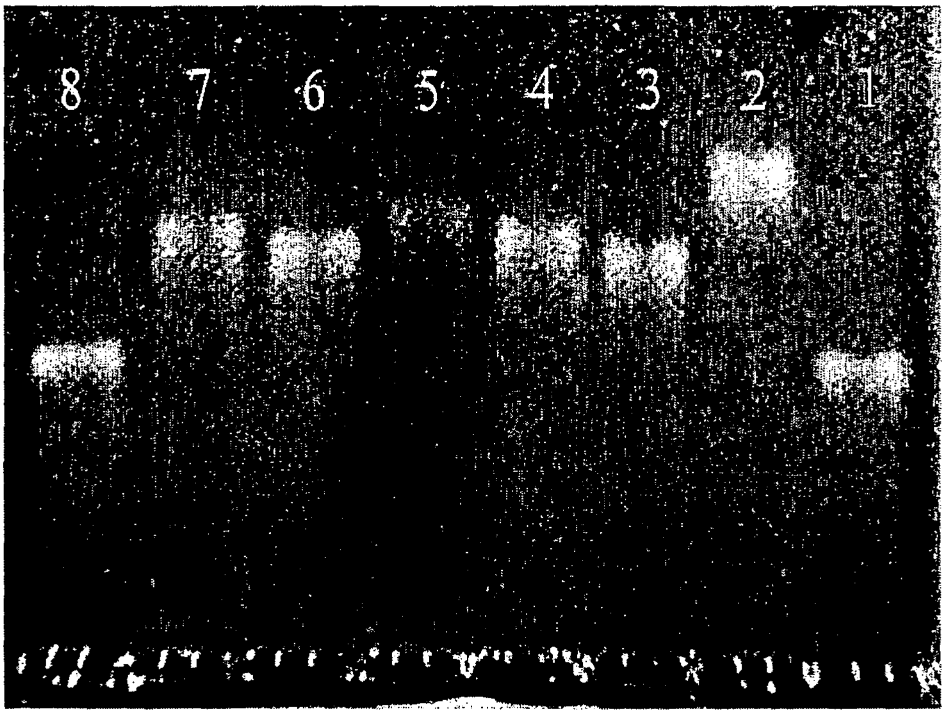

Figure 9

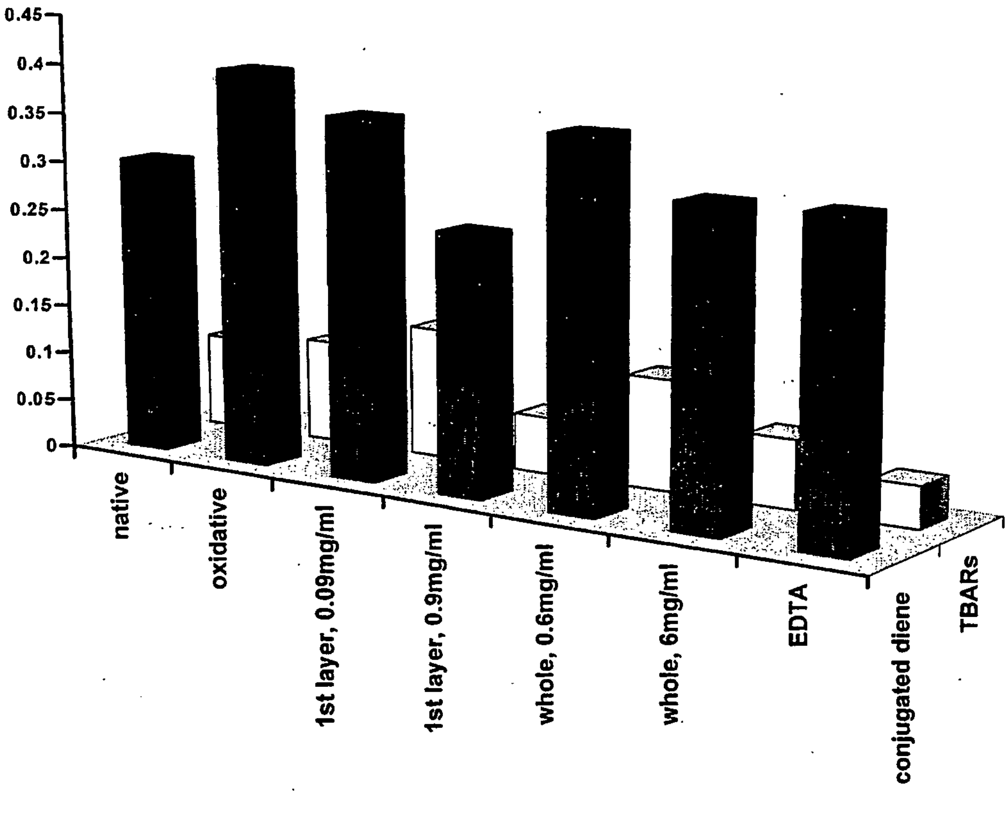
0.45
0.4
0.35
0.3
0.25
0.2
0.15
0.1
0.05
0
native
oxidative
1st layer, 0.09mg/ml
1st layer, 0.9mg/ml
whole, 0.6mg/ml
whole, 6mg/ml
EDTA
conjugated diene
TBARs

Figure 10

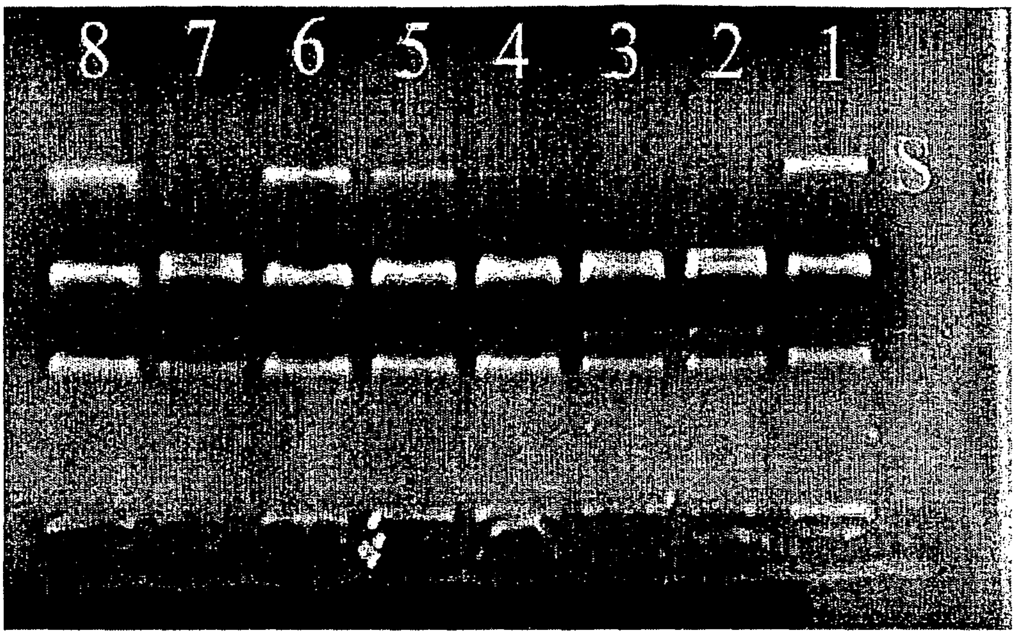

Figure 11

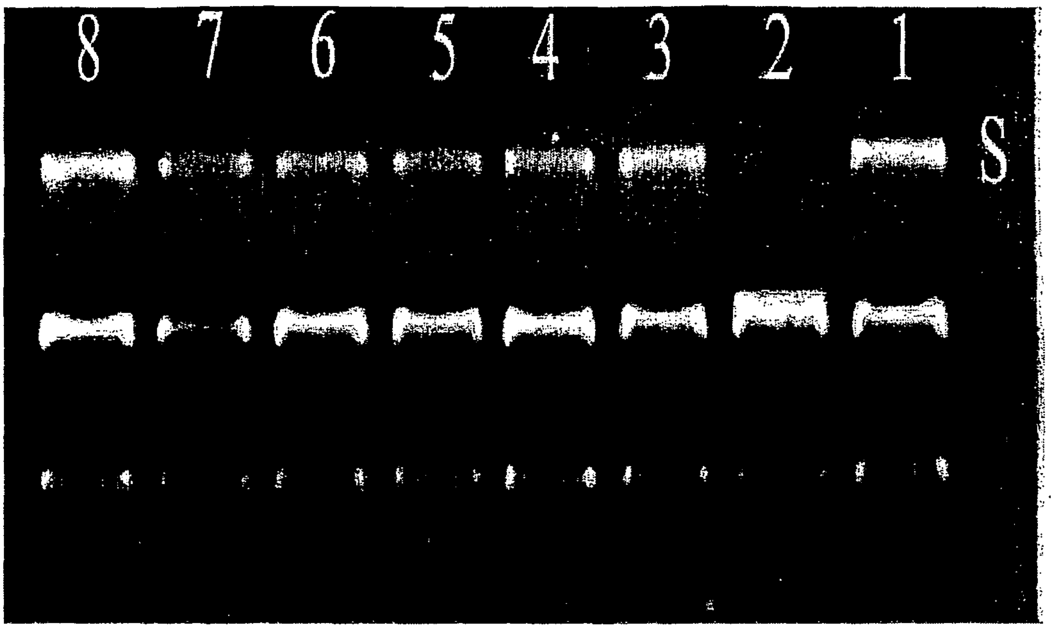

Figure 12

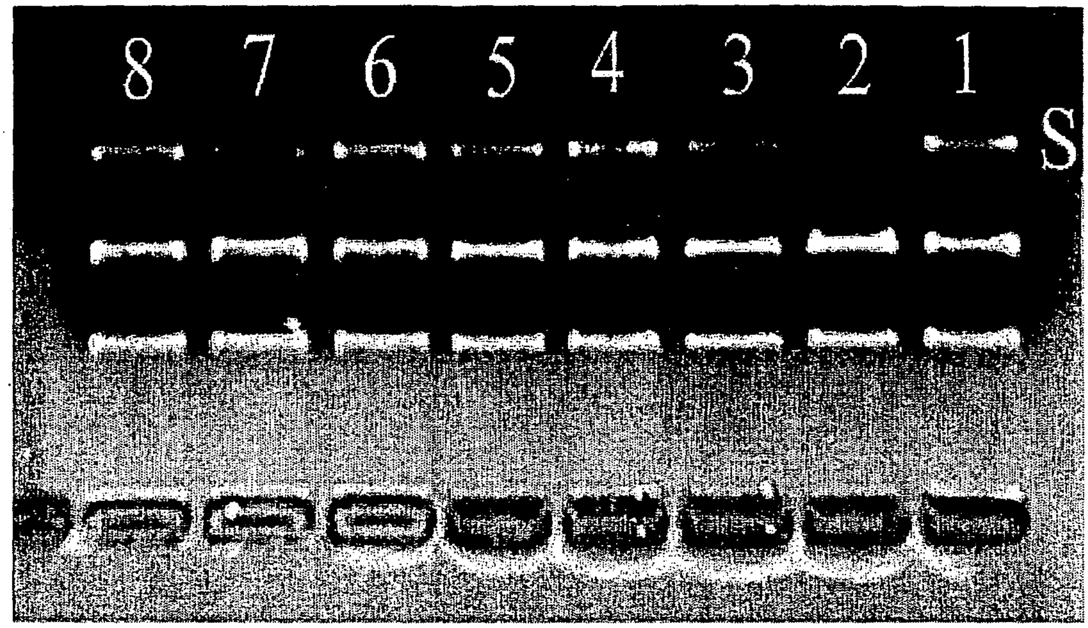

Figure 13

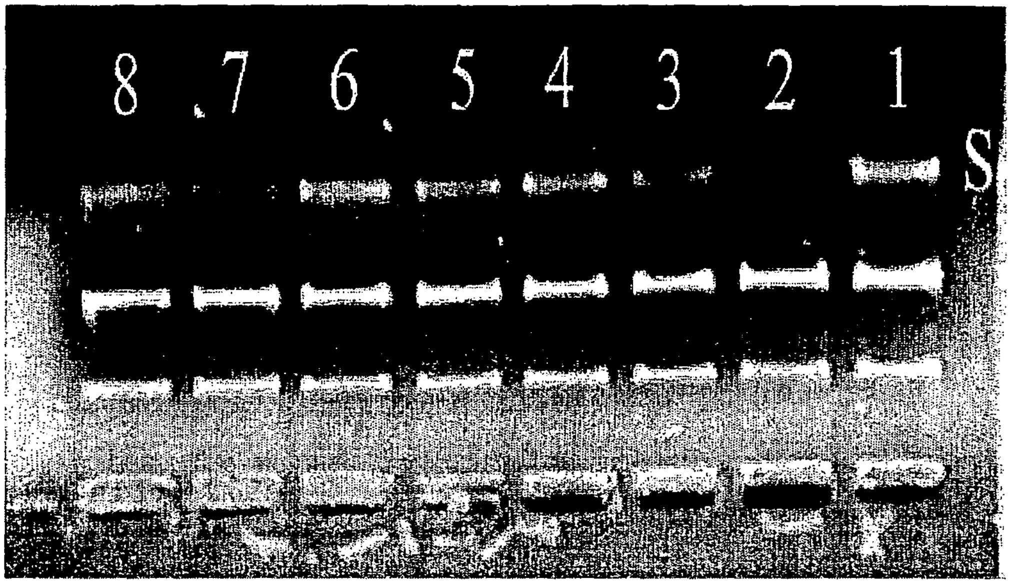

Figure 14

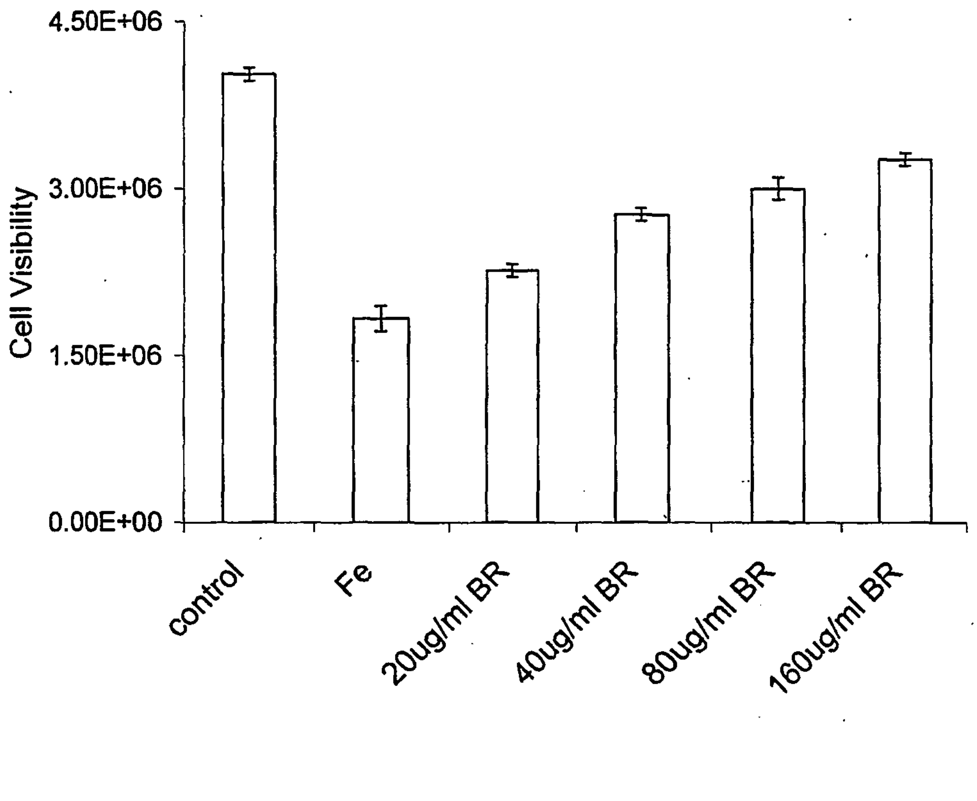
4.50E+06
3.00E+06
1.50E+06
0.00E+00
Cell Visibility
control
Fe
20ug/ml BR
40ug/ml BR
80ug/ml BR
160ug/ml BR

Figure 15

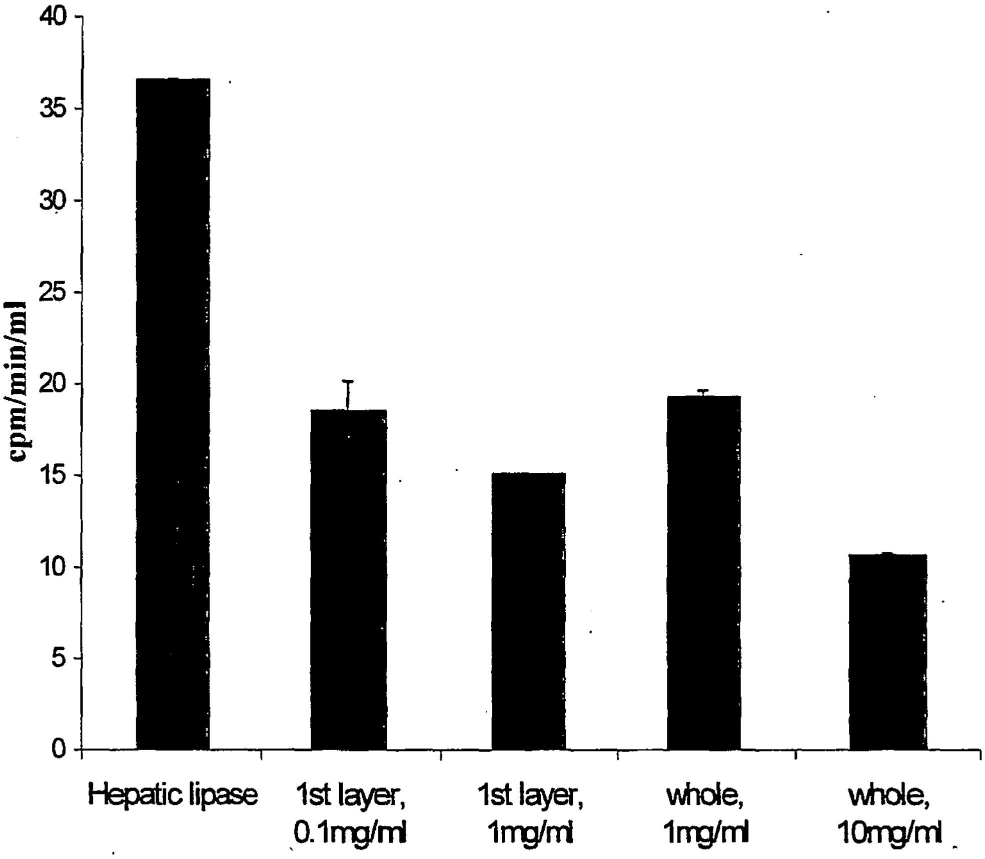
cpm/min/ml
40
35
30
25
20
15
10
5
0
Hepatic lipase
1st layer, 0.1mg/ml
1st layer, 1mg/ml
whole, 1mg/ml
whole, 10mg/ml

Figure 16

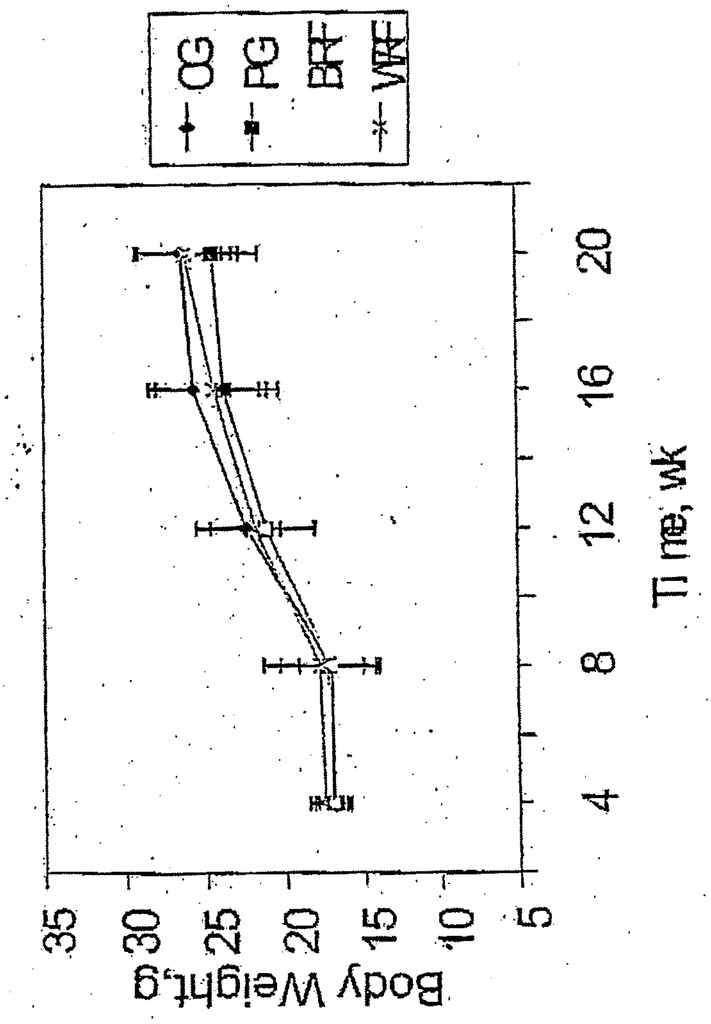

Figure 17

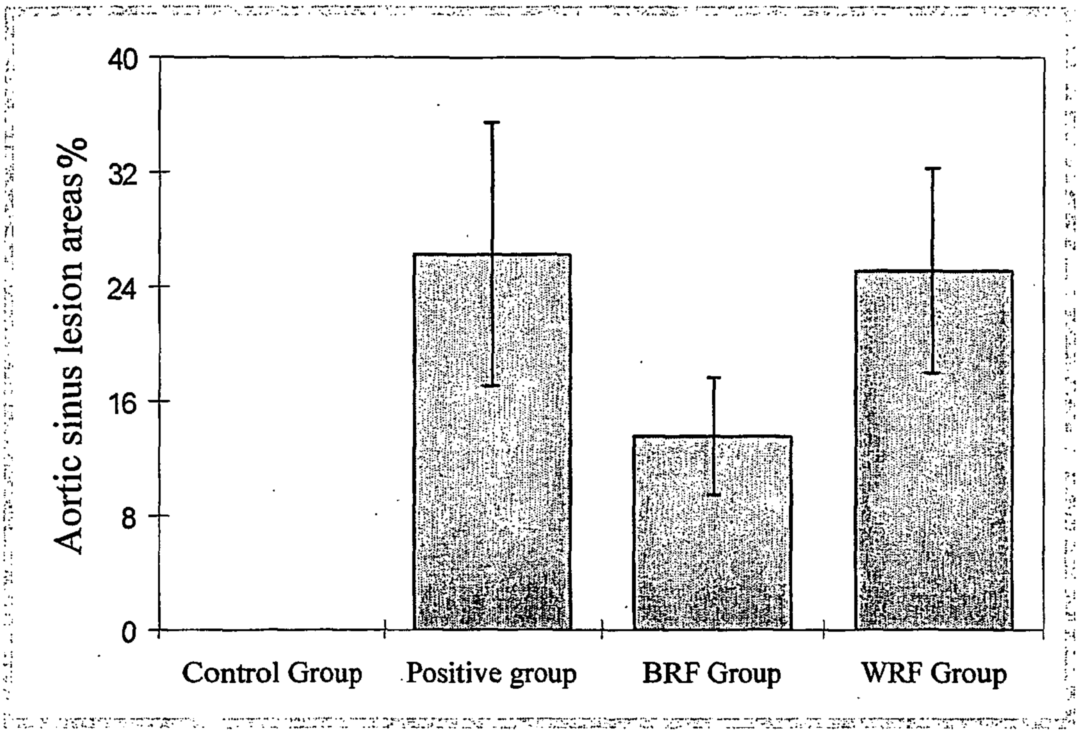
Aortic sinus lesion areas%
40
32
24
16
8
0
Control Group
Positive group
BRF Group
WRF Group

Figure 18

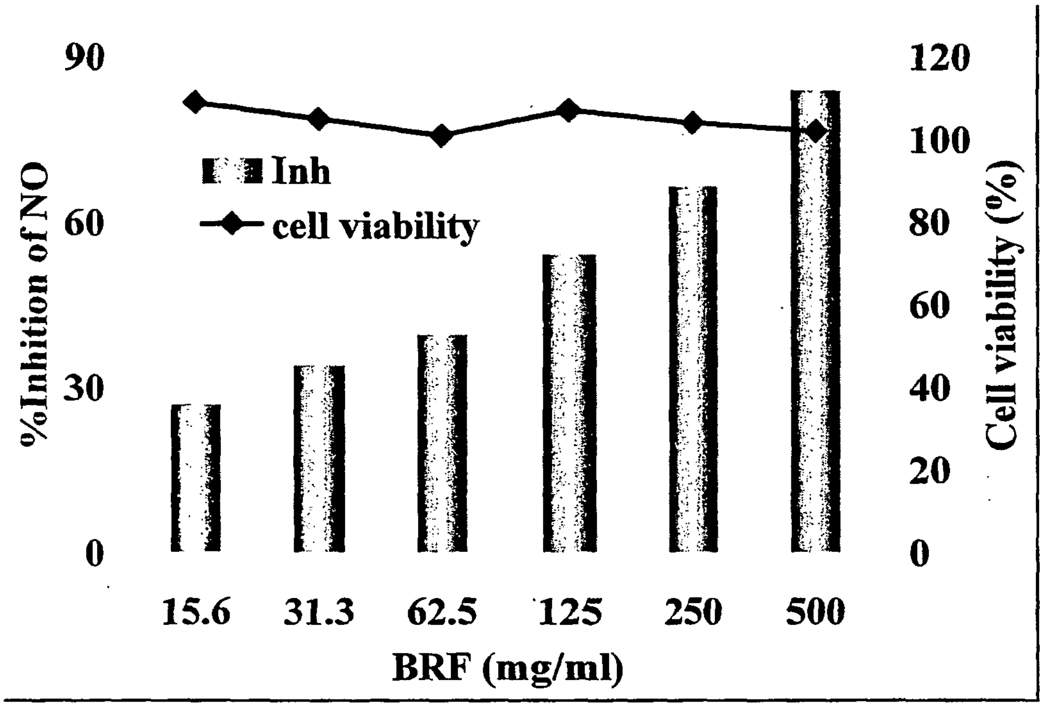
90
60
30
0
%Inhition of NO
120
100
80
60
40
20
0
Cell viability (%)
Inh
cell viability
15.6
31.3
62.5
125
250
500
BRF (mg/ml)

Figure 19

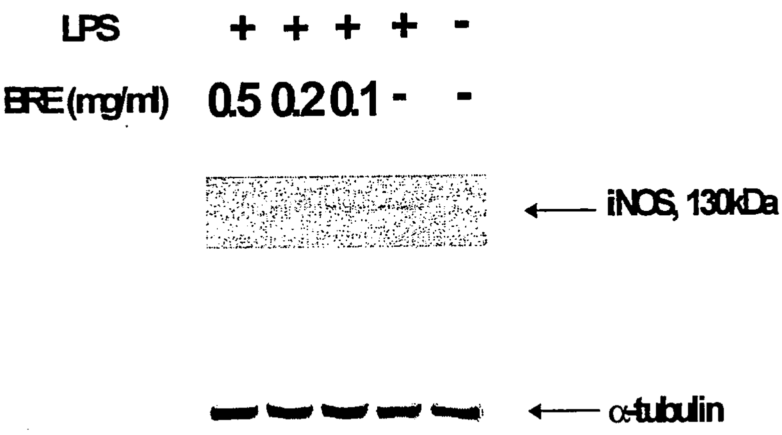

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 93109627
- US 4420427 A
- US 5770749 A


### Non-patent literature cited in the description


- **Su Noh Ryu et al.** High performance liquid chromatographic determination of anthocyanin pigments in some varieties of black rice. *Journal of Food and Drug Analysis,* 1998, vol. 6 (4), 729-736
- **W.H. Ling et al.** Red and black rice decrease atheroslerotic plaque formation and increase antioxidant status in rabbits. *J. Nutr.,* 2001, vol. 131, 1421-1426
- **Breslow.** *Nat.Med,* 1997, vol. 3, 600-601
- **Law et al.** *Br. Med. J.,* 1994, vol. 308, 363-366
- **Law et al.** *Br. Med. J.,* 1994, vol. 308, 367-373
- *Circulation,* 1990, vol. 81, 1721-1733
- **Havel R.J. ; Rapaport E.** *New England Journal of Medicine,* 1995, vol. 332, 1491-1498
- **Barker ; Rye.** *Atherosclerosis,* 1996, vol. 121, 1-12
- **Braunwald E.** *N. Engl. J. Med.,* 1997, vol. 340, 115-126
- **Ross R.** *N. Engl. J. Med.,* 1999, vol. 340, 115-126
- **Liao D.F. et al.** *J. Biol. Chem.,* 2000, vol. 275, 189-196
- **Shackelford R.E. et al.** *Free Rad. Biol. Med.,* 2000, vol. 28, 1387-1404
- **Giugliano D.** *Nutr. Metab. Cardiovasc. Dis.,* 2000, vol. 10, 38-44
- **Kunsch C. ; Medford R.M.** *Circ. Res.,* 1999, vol. 85, 753-766
- **Yuan, Y.V. ; Kitts, D.D. ; Godin, D.V.** Influence of dietary cholesterol and fat source on atherosclerosis in Japanese quail. *Brit. J. Nutr.,* 1997, vol. 78, 993-1014
- **Genest J.** *Can. J. Physiol. Pharmacol.,* 1986, vol. 64, 836-839
- **Simmons D. ; Willaims R.** *Br. J. Nutr.,* 1997, vol. 78, 5-14
- **Ling et al.** *Biomedical and Molecular Action of Nutrients,* 2001
- Liposome Drug Delivery Systems. Technomic Publishing Co. Inc, 1993
- *Pharmaceutical Technology: Liposomes as Drug Delivery Systems Parts I , II and III,* October 1992
- **Paigen B et al.** *Atherosclerosis,* 1987, vol. 68, 231
- **Schneider, J.E. ; Browning, M.M. ; Floyd, R.A.** Ascorbate/iron mediation of hydroxyl free radical damage to PBR322 plasmid DNA. *Free Rad Biol Med,* 1988, vol. 5, 287-295
- **Kitts, D.D. ; Yuan, Y.V. ; Wijewickreme, A.N. ; Thompson, L.U.** Antioxidant activity of the flaxseed lignan secoislariciresinol diglycoside and its mammalian lignan metabolites enterodiol and enterolactone. *Mol Cell Biochem,* 1999, vol. 202, 91-101
- **Keefer, L.K. ; Wink, K.** DNA damage and nitric oxide. *Adv Exp Med Biol.,* 2002, vol. 387, 177-185
- **Leeuwenburgh, C. ; Hardy, M.M. ; Hazen, S.L. ; Wagner, P. ; Oh-ishi, S. ; Steinbrecher, U.P. ; Heinecke, J.** Reactive nitrogen intermediates promote low density lipoprotein oxidation in human atherosclerotic initima. *J Biol Chem,* 1997, vol. 272, 1433-1438